# EUROPEAN PATENT APPLICATION

(11) **EP 3 677 596 A1**
(43) Date of publication of application: **08.07.2020**
(21) Application number: 18852415.1
(22) Date of filing: 29.08.2018
(51) Int. Cl.: C07K 16/24, A61K 9/00, A61K 47/14, A61K 47/26, A61K 47/18, A61K 39/00

(54) **METHOD FOR TREATING TNF -RELATED DISEASE**

(30) Priority: 30.08.2017 KR 20170110426; 01.11.2017 KR 20170144521; 13.02.2018 KR 20180017449
(71) Applicant: Celltrion Inc., Incheon 22014 (KR)
(72) Inventor: KIM, Sun Jung, Incheon 22014 (KR); SUH, Jee Hye, Incheon 22014 (KR); AN, Hyun Chul, Incheon 22014 (KR); LEE, Sung Young, Incheon 22014 (KR)
(74) Representative: D Young & Co LLP
(86) International application number: PCT/KR2018/009998
(87) International publication number: WO 2019/045452

(57) **Abstract**

The present invention relates to a method of TNF-α-related disease by subcutaneously administering an antibody binding to TNF-α (anti-TNF-α antibody). A treatment method, composition, kit or use according to the present invention reduces the time for administration and the time for patients to stay in hospitals, thereby improving patient convenience and the quality of life of the patient. This provides the advantage of improving the patient's satisfaction.

<110>    CELLTRION, INC.

<120>    Methods for treating TNFa-related diseases

<130>    CT-P13

<150>    KR 10-2017-0110426
<151>    2017-08-30

<150>    KR 10-2017-0144521
<151>    2017-11-01

<150>    KR 10-2018-0017449
<151>    2018-02-13

<160>    10

<170>    KoPatentIn 3.0

<210>    1
<211>    6
<212>    PRT
<213>    Artificial Sequence

<220>
<223>    Antibody

<400>    1
Gln Phe Val Gly Ser Ser
  1               5

<210>    2
<211>    3
<212>    PRT
<213>    Artificial Sequence

<220>
<223>    Antibody

<400>    2
Tyr Ala Ser
  1

<210>    3
<211>    9
<212>    PRT
<213>    Artificial Sequence

<220>
<223>    Antibody

<400>    3
Gln Gln Ser His Ser Trp Pro Phe Thr
  1               5

<210>    4
<211>    8

<212>     PRT
<213>     Artificial Sequence

<220>
<223>     Antibody


<400>     4
Gly Phe Ile Phe Ser Asn His Trp
1               5


<210>     5
<211>     10
<212>     PRT
<213>     Artificial Sequence

<220>
<223>     Antibody


<400>     5
Ile Arg Ser Lys Ser Ile Asn Ser Ala Thr
1               5                   10


<210>     6
<211>     11
<212>     PRT
<213>     Artificial Sequence

<220>
<223>     Antibody


<400>     6
Ser Arg Asn Tyr Tyr Gly Ser Thr Tyr Asp Tyr
1               5                   10


<210>     7
<211>     107
<212>     PRT
<213>     Artificial Sequence

<220>
<223>     Antibody


<400>     7
Asp Ile Leu Leu Thr Gln Ser Pro Ala Ile Leu Ser Val Ser Pro Gly
1               5                   10                  15

Glu Arg Val Ser Phe Ser Cys Arg Ala Ser Gln Phe Val Gly Ser Ser
            20                  25                  30

Ile His Trp Tyr Gln Gln Arg Thr Asn Gly Ser Pro Arg Leu Leu Ile
        35                  40                  45

Lys Tyr Ala Ser Glu Ser Met Ser Gly Ile Pro Ser Arg Phe Ser Gly
    50                  55                  60

Ser Gly Ser Gly Thr Asp Phe Thr Leu Ser Ile Asn Thr Val Glu Ser

65                  70                  75                  80

Glu Asp Ile Ala Asp Tyr Tyr Cys Gln Gln Ser His Ser Trp Pro Phe
                85                  90                  95

Thr Phe Gly Ser Gly Thr Asn Leu Glu Val Lys
            100                 105

<210>  8
<211>  119
<212>  PRT
<213>  Artificial Sequence

<220>
<223>  Antibody

<400>  8
Glu Val Lys Leu Glu Glu Ser Gly Gly Gly Leu Val Gln Pro Gly Gly
1               5                   10                  15

Ser Met Lys Leu Ser Cys Val Ala Ser Gly Phe Ile Phe Ser Asn His
            20                  25                  30

Trp Met Asn Trp Val Arg Gln Ser Pro Glu Lys Gly Leu Glu Trp Val
        35                  40                  45

Ala Glu Ile Arg Ser Lys Ser Ile Asn Ser Ala Thr His Tyr Ala Glu
    50                  55                  60

Ser Val Lys Gly Arg Phe Thr Ile Ser Arg Asp Asp Ser Lys Ser Ala
65                  70                  75                  80

Val Tyr Leu Gln Met Thr Asp Leu Arg Thr Glu Asp Thr Gly Val Tyr
                85                  90                  95

Tyr Cys Ser Arg Asn Tyr Tyr Gly Ser Thr Tyr Asp Tyr Trp Gly Gln
            100                 105                 110

Gly Thr Thr Leu Thr Val Ser
        115

<210>  9
<211>  236
<212>  PRT
<213>  Artificial Sequence

<220>
<223>  Antibody

<400>  9
Met Asp Phe Gln Val Gln Ile Ile Ser Phe Leu Leu Ile Ser Ala Ser
1               5                   10                  15

Val Ile Met Ser Arg Gly Asp Ile Leu Leu Thr Gln Ser Pro Ala Ile
            20                  25                  30

Leu Ser Val Ser Pro Gly Glu Arg Val Ser Phe Ser Cys Arg Ala Ser
        35                  40                  45

Gln Phe Val Gly Ser Ser Ile His Trp Tyr Gln Gln Arg Thr Asn Gly
    50                  55                  60

Ser Pro Arg Leu Leu Ile Lys Tyr Ala Ser Glu Ser Met Ser Gly Ile
65                  70                  75                  80

Pro Ser Arg Phe Ser Gly Ser Gly Ser Gly Thr Asp Phe Thr Leu Ser
                85                  90                  95

Ile Asn Thr Val Glu Ser Glu Asp Ile Ala Asp Tyr Tyr Cys Gln Gln
            100                 105                 110

Ser His Ser Trp Pro Phe Thr Phe Gly Ser Gly Thr Asn Leu Glu Val
        115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu
                165                 170                 175

Gln Ser Gly Asn Ser Gln Glu Ser Val Thr Glu Gln Asp Ser Lys Asp
            180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys
225                 230                 235


<210>   10
<211>   119
<212>   PRT
<213>   Artificial Sequence

<220>
<223>   Antibody


<400>   10
Glu Val Lys Leu Glu Glu Ser Gly Gly Gly Leu Val Gln Pro Gly Gly
1               5                   10                  15

Ser Met Lys Leu Ser Cys Val Ala Ser Gly Phe Ile Phe Ser Asn His
            20                  25                  30

Trp Met Asn Trp Val Arg Gln Ser Pro Glu Lys Gly Leu Glu Trp Val
        35                  40                  45

Ala Glu Ile Arg Ser Lys Ser Ile Asn Ser Ala Thr His Tyr Ala Glu
    50                  55                  60

Ser Val Lys Gly Arg Phe Thr Ile Ser Arg Asp Asp Ser Lys Ser Ala
65                  70                  75                  80

Val Tyr Leu Gln Met Thr Asp Leu Arg Thr Glu Asp Thr Gly Val Tyr

                85                  90                  95

Tyr Cys Ser Arg Asn Tyr Tyr Gly Ser Thr Tyr Asp Tyr Trp Gly Gln
            100                 105                 110

Gly Thr Thr Leu Thr Val Ser
        115

## Description

### [Technical Field]

This application relates to a method of TNF-α-related disease by subcutaneously administering an antibody binding to TNF-α (anti-TNF-a antibody).

### [Background Art]

Tumor necrosis factor-α (TNF-α) is a cell signaling protein (cytokine) that is involved in systemic inflammation and is a cytokine that mediates acute-phase responses. TNF-α is related to various diseases and disorders, including septicemia, infection, autoimmune diseases, and graft rejection. TNF-α stimulates immune responses and causes many clinical problems associated with autoimmune abnormalities such as rheumatoid arthritis, ankylosing spondylitis, ulcerative colitis, adult Crohn's disease, pediatric Crohn's disease, psoriasis, psoriatic arthritis and the like. Such abnormalities may be treated using TNF-α inhibitors.

Infliximab is a type of chimeric monoclonal antibody capable of acting as the TNF-α inhibitor, and currently commercially available infliximab products include Remsima, Remicade, Renflexis and the like. However, these products are all provided as lyophilized powders, which are reconstituted and diluted, and injected intravenously in a dosage regimen and dose selected according to each disease.

However, the intravenous administration method as described above requires the patient to visit the hospital for medication and takes 2 to 4 hours including the waiting time, indicating that it poses a considerable burden and inconvenience in daily life. In addition, there is a problem that a person who administer the drug is limited to a person who received medical education.

Therefore, subcutaneous (SC) administration is proposed as an alternative route of administration. Subcutaneous administration can be self-injected by a trained patient and can shorten the administration time from 30-90 minutes in a conventional art to 2-5 minutes.

Commercially available formulation products developed not only for intravenous administration, but also for subcutaneous administration, Rituxan (Rituximab), Simponi (Golimumab), Herceptin (Trastuzumab), Actemra (Tocilizumab), Xolair (Omalizumab), and the like, but a formulation for subcutaneous administration of Infliximab has not yet been reported.

For subcutaneous administration, a stable liquid formulation containing a high concentration of antibody is required, and the clinical efficacy and safety thereof should be demonstrated.

The Applicant has demonstrated the efficacy and stability of an Infliximab formulation for subcutaneous administration, which are equivalent to those of conventional formulations for intravenous administration, thereby completing a subcutaneous administration regimen that improves patient convenience and improves the quality of life of the patient.

### [Disclosure]

### [Technical Problem]

It is an object of the present invention to provide a treatment method comprising administering subcutaneously to a subject a pharmaceutical composition containing an anti-TNF-a antibody or its antigen binding fragment for treatment of TNF-α-related disease.

Another object of the present invention is to provide a pharmaceutical composition for treatment of a disease treatable with an anti-TNF-a antibody, which contains the anti-TNF-a antibody or its antigen binding fragment and is to be administered subcutaneously to a subject.

Still another object of the present invention is to provide a kit comprising: a pharmaceutical composition containing an anti-TNF-a antibody or its antigen binding fragment; and instructions that direct the pharmaceutical composition to be administered subcutaneously to a subject in order to treat disease treatable with the anti-TNF-a antibody.

Yet another object of the present invention is to provide the use of an anti-TNF-a antibody or its antigen binding fragment in the manufacture of a medicament which is to be administered subcutaneously to a subject in order to treat a disease treatable with the anti-TNF-a antibody.

### [Technical Solution]

The present invention provides a method for treating disease treatable with an anti-TNF-a antibody, the method comprising a step of administering subcutaneously to a subject a pharmaceutical composition containing the anti-TNF-a antibody or its antigen binding fragment.

The present invention also provides a pharmaceutical composition for treatment of a disease treatable with an anti-TNF-α antibody, which contains the anti-TNF-a antibody or its antigen binding fragment and is to be administered subcutaneously to a subject.

The present invention also provides a kit comprising: (a) a pharmaceutical composition containing an anti-TNF-a antibody or its antigen binding fragment, and pharmaceutically acceptable carrier; and (b) instructions that direct the pharmaceutical composition to be administered subcutaneously to a subject in order to treat a disease treatable with the anti-TNF-α antibody.

The present invention also provides the use of an anti-TNF-α antibody or its antigen binding fragment in the preparation of a pharmaceutical composition which is to be administered subcutaneously to a subject in order to treat a disease treatable with the anti-TNF-a antibody.

In one embodiment of the present invention, the anti-TNF-α antibody may comprise one or more selected from the group consisting of infliximab, adalimumab, certolizumab pegol, golimumab, and biosimilar thereof.

In one embodiment of the present invention, the anti-TNF-α antibody may be infliximab.

In one embodiment of the present invention, the anti-TNF-α antibody may comprise a chimeric human-mouse IgG monoclonal antibody.

In one embodiment of the present invention, the anti-TNF-α antibody may comprise: a light-chain variable region comprising a CDR1 domain comprising an amino acid sequence of SEQ ID NO: 1, a CDR2 domain comprising an amino acid sequence of SEQ ID NO: 2, and a CDR3 domain comprising an amino acid sequence of SEQ ID NO: 3; and a heavy-chain variable region comprising a CDR1 domain comprising an amino acid sequence of SEQ ID NO: 4, a CDR2 domain comprising an amino acid sequence of SEQ ID NO: 5, and a CDR3 domain comprising an amino acid sequence of SEQ ID NO: 6.

In one embodiment of the present invention, the anti-TNF-α antibody may comprise: a light-chain variable region comprising an amino acid sequence of SEQ ID NO: 7; and a heavy-chain variable region comprising an amino acid sequence of SEQ ID NO: 8.

In one embodiment of the present invention, the anti-TNF-α antibody may comprise: a light chain comprising an amino acid sequence of SEQ ID NO: 9; and a heavy chain comprising an amino acid sequence of SEQ ID NO: 10.

In one embodiment of the present invention, the composition may comprise: a surfactant; a sugar or its derivative; and a buffer comprising acetate or histidine.

In one embodiment of the present invention, the composition may comprise, as a surfactant, polysorbate 20, polysorbate 40, polysorbate 60, polysorbate 80, or a mixture thereof.

In one embodiment of the present invention, the concentration of the surfactant in the composition may be 0.02 to 0.1 % (w/v).

In one embodiment of the present invention, the composition may comprise, as sugar or its derivative, sorbitol, mannitol, trehalose, sucrose, or a mixture thereof.

In one embodiment of the present invention, the concentration of the sugar or its derivative in the composition may be 1 to 10 % (w/v).

In one embodiment of the present invention, the composition may comprise, as a buffer, acetate.

In one embodiment of the present invention, the concentration of the buffer in the composition may be 1 to 50 mM.

In one embodiment of the present invention, the composition may have a pH of 4.0 to 5.5.

In one embodiment of the present invention, the composition may comprise: (A) 90 to 180 mg/ml of the anti-TNF-a antibody or its antigen binding fragment; (B) 0.02 to 0.1 % (w/v) of polysorbate; (C) 1 to 10% (w/v) of sorbitol; and (D) 1 to 50 mM of a buffer comprising acetate or histidine.

In one embodiment of the present invention, the composition may be free of aspartic acid, lysine, arginine, or a mixture thereof.

In one embodiment of the present invention, the composition may be free of NaCl, KCl, NaF, KBr, NaBr, Na₂SO₄, NaSCN, K₂SO₄, or a mixture thereof.

In one embodiment of the present invention, the composition may be free of a chelating agent.

In one embodiment of the present invention, the composition may have a viscosity of 0.5 cp to 10.0 cp after 1 month of storage at a temperature of 40°C ± 2°C, or a viscosity of 0.5 cp to 5 cp after 6 months of storage at a temperature of 5°C ± 3°C.

In one embodiment of the present invention, the composition may not be subjected to a reconstitution step, a dilution step, or both, before use.

In one embodiment of the present invention, the composition may be filled in a pre-filled syringe or an auto-injector before administration to the subject.

In one embodiment of the present invention, the subject may include mammals.

In one embodiment of the present invention, the subject may include human beings.

In one embodiment of the present invention, the antibody or its antigen binding fragment may be administered at a dose of 60 to 300 mg.

In one embodiment of the present invention, the antibody or its antigen binding fragment may be administered at a dose of 60, 70, 80, 90, 100, 110, 120, 130, 140, 150, 160, 170, 180, 190, 200, 210, 220, 230, 240, 250, 260, 270, 280, 290 or 300 mg.

In one embodiment of the present invention, the antibody or its antigen binding fragment may be administered at a dose of 90 to 180 mg.

In one embodiment of the present invention, the antibody or its antigen binding fragment may be administered at a dose of 120 to 240 mg.

In one embodiment of the present invention, the antibody or its antigen binding fragment may be administered at a dose of 80 to 100 mg, 110 to 130 mg, 170 to 190 mg, or 230 to 250 mg.

In one embodiment of the present invention, the antibody or its antigen binding fragment may be administered at a dose of 90 mg, 120 mg, 180 mg or 240 mg.

In one embodiment of the present invention, the antibody or its antigen binding fragment may be administered at a dose of 90 to 180 mg when the body weight of the patient is less than 80 kg, and may be administered at a dose of 190 to 270 mg when the body weight of the patient is more than 80 kg.

In one embodiment of the present invention, the antibody or its antigen binding fragment may be administered at intervals of 1 to 8 weeks.

In one embodiment of the present invention, the antibody or its antigen binding fragment may be administered at intervals of 1, 2, 3, 4, 5, 6, 7 or 8 weeks.

In one embodiment of the present invention, the antibody or its antigen binding fragment may be administered at intervals of 2 or 4 weeks.

In one embodiment of the present invention, the diseases treatable with the anti-TNF-a antibody may include rheumatoid arthritis, ulcerative colitis, Crohn's disease, plaque psoriasis, psoriatic arthritis, and ankylosing spondylitis.

In one embodiment of the present invention, the patient to be administered with the anti-TNF-a antibody may exhibit one or more characteristics selected from the following:
a) a patient who has an inadequate response to disease-modifying anti rheumatic drugs (DMARDs), including methotrexate;
b) a patient who has not previously been treated with methotrexate and other DMARDs;
c) a patient who exhibits elevated serologic indicators associated with severe axial-predominant symptoms and inflammation, which show no proper response to common therapies;
d) a patient who does not respond to, or is contraindicated to, or has intolerance to methotrexate, cyclosporine, or systemic therapies including psoralen ultraviolet A therapy (PUVA);
e) a patient who has an inadequate response to, or has intolerance to, or is contraindicated for treatment with corticosteroids, 6-mercaptopurine, azathioprine or immunosuppressants; and
f) a patient who does not respond to common therapies, including antibiotic, excretion or immunosuppressive therapies.

In one embodiment of the present invention, the patient may be a patient who has been administered at least once intravenously with the anti-TNF-a antibody or its antigen binding fragment prior to subcutaneous administration.

In one embodiment of the present invention, the patient may be a patient who has been administered intravenously with the anti-TNF-a antibody or its antigen binding fragment at a dose of 1 to 10 mg/kg for each administration prior to subcutaneous administration.

In one embodiment of the present invention, the first subcutaneous administration may be performed 2 to 8 weeks after the last intravenous administration.

In one embodiment of the present invention, the first subcutaneous administration may be performed 4 weeks after the last intravenous administration.

In one embodiment of the present invention, the composition containing the anti-TNF-a antibody or its antigen binding fragment may be administered simultaneously with, before or after administration of one or more selected from the group consisting of infliximab, adalimumab, certolizumab pegol, golimumab, and biosimilar thereof.

In one embodiment of the present invention, the composition containing the anti-TNF-a antibody or its antigen binding fragment may be administered simultaneously with, before or after administration of methotrexate, lefuromide and sulfasalazine, hydroxychloroquine, or a mixture thereof.

In one embodiment of the present invention, the patient after subcutaneous administration may exhibit one or more characteristics selected from the following:
a) a decrease in DAS28 (Disease Activity Score in 28 joints) of at least 2.0; and
b) a decrease in CDAI (Crohn's disease activity index) of at least 70.

### [Advantageous Effects]

The treatment method, composition, kit or use according to the present invention makes it possible to treat TNF-α-related disease by subcutaneously administering the anti-TNF-a antibody or its antigen binding fragment. In addition, the treatment method, composition, kit or use according to the present invention reduces the time for administration and the time for patients to stay in hospitals, thereby improving patient convenience and the quality of life of the patient. This provides the advantage of improving the patient's satisfaction.

In addition, the treatment method, composition, kit or use according to the present invention is added as a new treatment option of infliximab, with the result that patients who have been administered intravenously with infliximab, as well as healthcare workers, do not have the burden and rejection caused by drug changes.

### [Description of Drawings]

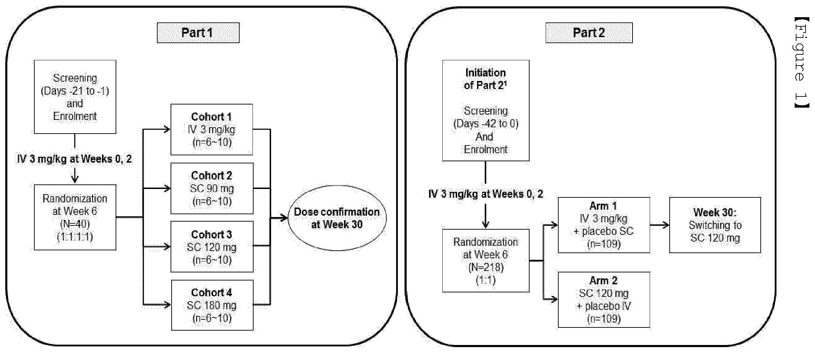

FIG. 1 schematically shows a design of a clinical test for subcutaneous administration of infliximab to rheumatoid arthritis (RA) patients.

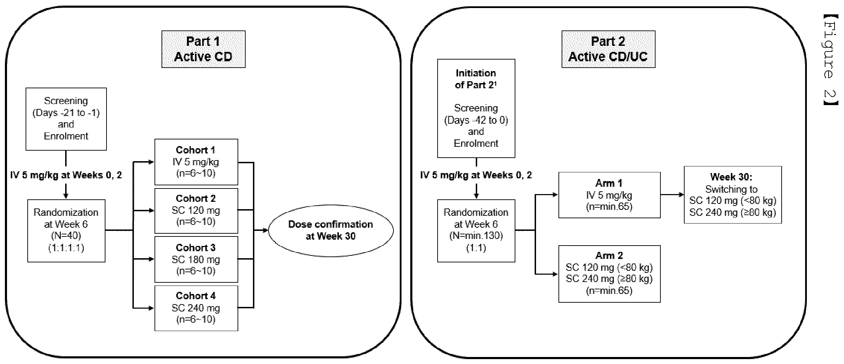

FIG. 2 schematically shows a design of a clinical test for subcutaneous administration of infliximab to Crohn's disease (CD) patients.

### [Mode for Invention]

The present invention is directed to a method for treatment of a disease treatable with an anti-TNF-a antibody, the method comprising a step of administering subcutaneously to a subject a pharmaceutical composition containing anti-TNF-a antibody or its antigen binding fragment.

To facilitate the understanding of the present invention, the terms used in the present invention are defined as follows.

"TNF-α" is intended to refer to a human cytokine that exists as a 17 kD secreted form and a 26 kD membrane associated form, the biologically active form of which is composed of a trimer of noncovalently bound 17 kD molecules. The structure of TNF-α is described further in, for example, Pennica, D., et al. (1984) Nature 312:724-729; Davis, J.M., et al. (1987) Biochemistry 26:1322-1326; and Jones, E.Y., et al. (1989) Nature 338:225-228.

The term "antibody" refers to immunoglobulin molecules comprised of four polypeptide chains, two heavy chains and two light chains inter-connected by disulfide bonds. Other naturally occurring antibodies having an altered structure, for example, camelid antibodies, are also included in this definition. Each heavy chain is comprised of a heavy-chain variable region and a heavy-chain constant region. The heavy-chain constant region is comprised of three domains (CH1, CH2 and CH3). Each light chain is comprised of a light-chain variable region and a light-chain constant region. The light-chain constant region is comprised of one domain (CL). The heavy-chain variable region and the light-chain variable region can be further subdivided into regions of hypervariability, termed complementarity determining regions (CDR), interspersed with regions that are more conserved, termed framework regions (FR). Each of the heavy-chain variable region and the light-chain variable region is composed of three CDRs and four FRs, which are arranged from amino-terminus to carboxy-terminus in the following order: FR1, CDR1, FR2, CDR2, FR3, CDR3, FR4.

The term "antigen binding fragment" refers to one or more fragments of an antibody that retain the ability to specifically bind to an antigen. Examples of antigen binding fragments include, but are not limited to Fab, Fab', F(ab')2, Fv, and the like.

The term "biosimilar" refers to a biological product that is highly similar to an FDA-approved biological product (reference drug) and has no clinically meaningful differences in terms of pharmacokinetics, safety and efficacy from the reference product.

The term "administration" refers to administration of a substance (e.g., anti-TNF-a antibody) for achieving therapeutic purposes (e.g., TNF-α-related disease).

The term "TNF-α-related disease" refers to a local and/or systemic physiological disease where TNF-α is a primary mediator leading to the manifestation of the disease. The term "TNF-α-related disease", "disease treatable with anti-TNF-a" and "disease where the activity of TNF-α is harmful" are used interchangeably herein.

The term "subject" includes all humans or non-human animals. The term "non-human animals" includes, but is not limited to, vertebrates, such as non-human primates, sheep, dogs, cats, rabbits and ferrets, rodents, such as mice, rats and guinea pigs, bird species such as chickens, amphibian, and reptile. In a preferred embodiment, the subject is mammals, such as non-human primates, sheep, dogs, cats, rabbits, ferrets, or rodents. In a more preferred embodiment, the subject is a human being. The terms "subject", "patient" and "individual" are used interchangeably herein.

The term "IC50" is intended to refer to the concentration of an inhibitor, which is required to inhibit the biological outcome of interest, for example, to neutralize cytotoxic activity.

The term "kit" refers to a packaged product comprising components for administrating the TNF-α antibody of the present invention to treat TNF-α-related disease. The kit preferably comprises a box or container that holds the components of the kit. The box or container is affixed with a label or a Food and Drug Administration approved protocol. The box or container holds components of the present invention that are contained within plastic, polyethylene, polypropylene, ethylene or propylene containers. The containers can be capped-tubes or bottles. The kit can also include instructions for administering the anti-TNF-a antibody.

Various aspects of the present invention will be described in further detail.

### - Anti-TNF-a antibody or its antigen binding fragment of the present invention

In one embodiment of the present invention, the pharmaceutical formulation may contain, as the antibody, a polyclonal antibody, a monoclonal antibody, a recombinant antibody, a single-chain antibody, a hybrid antibody, a chimeric antibody, a humanized antibody, or a fragment thereof. The term "chimeric antibody" refers to an antibody comprising heavy-chain and light-chain variable region sequences from one species and constant region sequences from another species. In one embodiment of the present invention, the pharmaceutical formulation may contain, as the antibody, a chimeric human-mouse IgG monoclonal antibody. The chimeric human-mouse IgG monoclonal antibody is comprised of mouse heavy-chain and light-chain variable regions and human heavy-chain and light-chain constant regions bound thereto. The chimeric human-mouse IgG monoclonal antibody may be produced according to a method known in the art. For example, infliximab may be produced according to a method described in US Patent No. 6,284,471.

In one embodiment of the present invention, the pharmaceutical formulation may contain, as the antibody, an antibody that binds to TNF-α or the epitope of TNF-α. The antibody that binds to TNF-α or the epitope of TNF-α may comprise infliximab, adalimumab, certolizumab pegol, golimumab, or biosimilar thereof. In one embodiment of the present invention, the antibody may comprise infliximab.

In one embodiment of the present invention, the antibody or its antigen-binding fragment (A) may comprise: a light-chain variable region comprising a CDR1 domain comprising an amino acid sequence of SEQ ID NO: 1, a CDR2 domain comprising an amino acid sequence of SEQ ID NO: 2, and a CDR3 domain comprising an amino acid sequence of SEQ ID NO: 3; and a heavy-chain variable region comprising a CDR1 domain comprising an amino acid sequence of SEQ ID NO: 4, a CDR2 domain comprising an amino acid sequence of SEQ ID NO: 5, and a CDR3 domain comprising an amino acid sequence of SEQ ID NO: 6.

In one embodiment of the present invention, the antibody or its antigen binding fragment (A) may comprise: a light-chain variable region having an amino acid sequence of SEQ ID NO: 7; and a heavy-chain variable region having an amino acid sequence of SEQ ID NO: 8.

In one embodiment of the present invention, the antibody or its antigen binding fragment (A) may comprise: a light chain having an amino acid sequence of SEQ ID NO: 9; and a heavy chain having an amino acid sequence of SEQ ID NO: 10.

### - Composition containing anti-TNF-α antibody or its antigen binding fragment of the present invention

As used herein, the expression "composition containing anti-TNF-a antibody or its antigen binding fragment of the present invention" is used interchangeably with "stable liquid pharmaceutical formulation".

A stable liquid pharmaceutical formulation according to the present invention contains: (A) an antibody or its antigen-binding fragment; (B) a surfactant; (C) a sugar or its derivative; and (D) a buffer.

As used herein, the term "free of" means that the formulation is completely free of the corresponding component. In addition, the term means that the formulation is substantially free of the corresponding component, that is, contains the corresponding component in an amount that does not affect the activity of the antibody and the stability and viscosity of the liquid pharmaceutical formulation. For example, the term means that the formulation contains the corresponding component in an amount of 0 to 1% (w/v), 0 to 1 ppm (w/v), or 0 to 1 ppb (w/v), based on the total weight of the liquid pharmaceutical formulation.

### (A) Antibody or Its Antigen-Binding Fragment

The concentration of the antibody or its antigen-binding fragment may be freely controlled within a range that does not substantially adversely affect the stability and viscosity of the stable liquid pharmaceutical formulation according to the present invention. In one embodiment of the present invention, the concentration of the antibody or its antigen-binding fragment may be 10 to 200 mg/ml. In another embodiment of the present invention, the concentration of the antibody or its antigen-binding fragment may be 50 to 200 mg/ml. In still another embodiment of the present invention, the concentration of the antibody or its antigen-binding fragment may be 80 to 150 mg/ml. In still another embodiment of the present invention, the concentration of the antibody or its antigen-binding fragment may be 90 to 145 mg/ml. In yet another embodiment of the present invention, the concentration of the antibody or its antigen-binding fragment may be 110 to 130 mg/ml. If the concentration of the antibody or its antigen-binding fragment is within the above-described range, the high content of the antibody or its antigen-binding fragment makes it possible to increase the degree of freedom of dose and administration cycle, and the pharmaceutical formulation may exhibit excellent long-term stability and low viscosity.

### (B) Surfactant

Examples of the surfactant include, but are not limited to, polyoxyethylene sorbitan fatty acid ester (e.g., polysorbate), polyoxyethylene alkyl ether (e.g., Brij), alkylphenyl polyoxyethylene ether (e.g., Triton-X), polyoxyethylene-polyoxypropylene copolymers (e.g., Poloxamer, Pluronic), sodium dodecyl sulfate (SDS), and the like.

In one embodiment of the present invention, the surfactant may comprise polyoxyethylene sorbitan fatty acid ester (polysorbate). The polysorbate may comprise polysorbate 20, polysorbate 40, polysorbate 60, polysorbate 80, or a mixture of two or more thereof. In one embodiment of the present invention, the polysorbate may comprise polysorbate 20, polysorbate 80, or a mixture thereof. In another embodiment of the present invention, the polysorbate may comprise polysorbate 80.

In one embodiment of the present invention, the concentration of the surfactant may be freely controlled within a range that does not substantially adversely affect the stability and viscosity of the stable liquid pharmaceutical formulation according to the present invention. For example, the concentration of the surfactant may be 0.001 to 5% (w/v), 0.01 to 1% (w/v), or 0.02 to 0.1% (w/v). If the concentration of the surfactant is within the above-described range, the pharmaceutical composition may exhibit excellent long-term stability and low viscosity.

### (C) Sugar or Its Derivative

The sugar may comprise a monosacchride, a disaccharide, an oligosaccharide, a polysaccharide, or a mixture of two or more thereof. Examples of the monosacchride include, but are not limited to, glucose, fructose, galactose, and the like. Examples of the disaccharide include, but are not limited to, sucrose, lactose, maltose, trehalose, and the like. Examples of the oligosaccharide include, but are not limited to, fructooligosaccaharides, galactooligosaccaharides, mannanoligosaccaharides, and the like. Examples of the polysaccharide include, but are not limited to, starch, glycogen, cellulose, chitin, pectin, and the like.

The sugar derivative may comprise sugar alcohol, sugar acid, or a mixture thereof. Examples of the sugar alcohol include, but are not limited to, glycerol, erythritol, threitol, arabitol, xylitol, ribitol, mannitol, sorbitol, galactitol, fucitol, iditol, inositol, volemitol, isomalt, maltitol, lactitol, maltotriitol, maltotetraitol, polyglycitol, and the like. Examples of the sugar acid include, but are not limited to, aldonic acid (glyceric acid, etc.), ulosonic acid (neuraminic acid, etc.), uronic acid (glucuronic acid, etc.), aldaric acid (tartaric acid, etc.), and the like.

In one embodiment of the present invention, the sugar or its derivative (C) may comprise sorbitol, mannitol, trehalose, sucrose, or a mixture of two or more thereof.

In one embodiment of the present invention, the concentration of the sugar or its derivative may be freely controlled within a range that does not substantially adversely affect the stability and viscosity of the stable liquid pharmaceutical formulation according to the present invention. For example, the concentration of the sugar or its derivative may be 0.1 to 30% (w/v), 1 to 20% (w/v), or 1 to 10% (w/v). If the concentration of the sugar or its derivative may be within this range, the pharmaceutical composition may exhibit excellent long-term stability and low viscosity.

### (D) Buffer

The buffer that is used in the present invention is a neutralizing substance that minimizes the change in pH caused by acid or alkali. Examples of the buffer include phosphate, acetate, succinate, gluconate, glutamate, citrate, histidine, and the like. In one embodiment of the present invention, the buffer may comprise acetate or histidine. If the buffer comprises both acetate and histidine, the stability of the pharmaceutical formulation may be reduced.

In one embodiment of the present invention, the buffer may comprise acetate. Examples of the acetate include, but are not limited to, sodium acetate, zinc acetate, aluminum acetate, ammonium acetate, potassium acetate, and the like. For pH adjustment, the buffer may further comprise an acid, for example, acetic acid. When the buffer comprises acetate, it may be most preferable in terms of pH adjustment and stability.

In one embodiment of the present invention, the buffer may comprise histidine. When the buffer comprises histidine, it may comprise a histidine salt, for example, histidine chloride, histidine acetate, histidine phosphate, histidine sulfate, or the like. For pH adjustment, the buffer may comprise an acid, for example, hydrochloric acid, acetic acid, phosphoric acid, sulfuric acid, or the like.

In one embodiment of the present invention, the stable liquid pharmaceutical formulation may be free of citrate, phosphate, or a mixture thereof.

In one embodiment of the present invention, the concentration of the buffer (or the anion of the buffer) may be freely controlled within a range that does not substantially adversely affect the stability and viscosity of the stable liquid pharmaceutical formulation according to the present invention. For example, the concentration of the buffer or its anion may be 1 to 50 mM, 5 to 30 mM, or 10 to 25 mM. If the concentration of the buffer or its anion is within this range, the pharmaceutical composition may exhibit excellent long-term stability and low viscosity.

### (E) pH

In one embodiment of the present invention, the pH of the stable liquid pharmaceutical composition may be 4.0 to 5.5, or 4.7 to 5.3. If the pH is within this range, the pharmaceutical composition may exhibit excellent long-term stability and low viscosity. The pH of the pharmaceutical formulation may be adjusted using the buffer. In other words, if the pharmaceutical formulation contains a certain content of the buffer, it may exhibit the pH in the above-described range without having to use a separate pH-adjusting agent. If citrate, phosphate or a mixture thereof is used as the buffer, it may be difficult to show the pH in the above-described range. If the pharmaceutical formulation further contains an acid (e.g., hydrochloric acid) or a base (e.g., sodium hydroxide) as a separate pH-adjusting agent, the stability of the antibody may be reduced.

### (F) Other Components

In one embodiment of the present invention, the stable liquid pharmaceutical formulation may be free of aspartic acid, lysine, arginine, or mixtures thereof. If the stable liquid pharmaceutical formulation contains these amino acids, it may become solid. In one embodiment of the present invention, the stable liquid pharmaceutical formulation may contain one or more amino acids, excluding the above-described three amino acids. In this case, the stable liquid pharmaceutical formulation may contain the one or more amino acid in an amount of 5% (w/v) or less, for example, 0.001 to 5% (w/v), 0.001 to 1% (w/v), 0.01 to 5% (w/v), 0.01 to 1% (w/v), 0.1 to 5% (w/v), or 0.1 to 1% (w/v).

In another embodiment of the present invention, the stable liquid pharmaceutical formulation may contain taurine. In this case, the taurine may be contained in an amount of 5% (w/v) or less, for example, 0.001 to 5% (w/v), 0.001 to 1% (w/v), 0.01 to 5% (w/v), 0.01 to 1% (w/v), 0.1 to 5% (w/v), or 0.1 to 1% (w/v).

In one embodiment of the present invention, the stable liquid pharmaceutical formulation may be free of a metal salt, such as NaCl, KCl, NaF, KBr, NaBr, Na₂SO₄, NaSCN, K₂SO₄ or the like. If the stable liquid pharmaceutical formulation contains these metal salts, precipitation in the formulation may occur, and the formulation may be gelatinized and may have poor stability.

In one embodiment of the present invention, the stable liquid pharmaceutical formulation may be free of a chelating agent (e.g., EDTA). If the pharmaceutical formulation contains a chelating agent, the oxidation rate thereof may be increased.

In one embodiment of the present invention, the stable liquid pharmaceutical formulation may be free of a preservative. Examples of the preservative include octadecyl dimethylbenzyl ammonium chloride, hexamethonium chloride, benzalkonium chloride, benzethonium chloride, phenol, butyl alcohol, benzyl alcohol, alkyl paraben, catechol, resorcinol, cyclohexanol, 3-pentanol, m-cresol, and the like. If the pharmaceutical formulation contains the preservative, the preservative may not help improve the stability of the pharmaceutical formulation.

In one embodiment of the present invention, the stable liquid pharmaceutical formulation of the present invention may further contain an additive known in the art, which does not substantially adversely affect the activity of the antibody and the stability and low viscosity of the formulation. For example, the pharmaceutical formulation may further contain an aqueous carrier, an antioxidant, or a mixture of two or more thereof. The aqueous carrier is a carrier that is pharmaceutically acceptable (safe and non-toxic when administered to humans) and is useful for preparation of liquid pharmaceutical formulations. Examples of the aqueous carrier include, but are not limited to, sterile water for injection (SWFI), bacteriostatic water for injection (BWFI), sterile saline solution, Ringer's solution, dextrose, and the like. Examples of the antioxidant include, but are not limited to, ascorbic acid and the like.

### (G) "Stable" Liquid Pharmaceutical Formulation

The term "stable" in the "stable" liquid pharmaceutical formulation of the present invention means that the antibody according to the present invention essentially retains its physical stability and/or chemical stability and/or biological activity during production and/or upon storage. Various analytical techniques for measuring protein stability are readily available in the art.

Physical stability may be assessed by methods known in the art, which include measurement of a sample's apparent attenuation of light (absorbance, or optical density). Such a measurement of light attenuation is related to the turbidity of a formulation. In addition, for physical stability, the contents of high-molecular-weight components, the contents of low-molecular-weight components, the amounts of intact proteins, the number of sub-visible particles, and the like, may be measured.

Chemical stability can be assessed by, for example, detecting and quantifying chemically altered forms of the antibody. Chemical stability includes charge alteration (for example, occurring as a result of deamidation or oxidation) which can be evaluated by, for example, ion-exchange chromatography. For chemical stability, charge variants (acidic or basic peaks) may be measured.

Biological activity may be assessed by methods known in the art. For example, antigen binding affinity may be measured by ELISA.

In one embodiment of the present invention, the liquid pharmaceutical formulation may be stable for a long period of time.

In one embodiment of the present invention, the term "stable" liquid pharmaceutical formulation means a liquid pharmaceutical formulation satisfying one or more of the following criteria.

### Turbidity

- a liquid pharmaceutical formulation having an absorbance A₆₀₀ of 0 to 0.0300, or 0 to 0.0700, as measured by a spectrophotometer after 4 weeks of storage at a temperature of 40°C ± 2°C;
- a liquid pharmaceutical formulation having an absorbance A₆₀₀ of 0 to 0.0300, or 0 to 0.0700, as measured by a spectrophotometer after 4 weeks of storage at a temperature of 40°C ± 2°C and a relative humidity of 75 ± 5% under a closed condition;
   **Content of Main Component** (main peak)
- a liquid pharmaceutical formulation in which the content of a main component content after 4 weeks of storage at a temperature of 40°C ± 2°C is 98% to 100% as measured by SE-HPLC;
- a liquid pharmaceutical formulation in which the content of a main component content after 4 weeks of storage at a temperature of 40°C ± 2°C and a relative humidity of 75 ± 5% under a closed condition is 98% to 100% as measured by SE-HPLC;
   **Content of High-Molecular-Weight Components** (a peak whose retention time is earlier than that of the main peak (intact IgG))
- a liquid pharmaceutical formulation in which the content of high-molecular-weight components after 12 months of storage at a temperature of 5°C ± 3°C is 0 to 1.00% as measured by SE-HPLC;
- a liquid pharmaceutical formulation in which the content of high-molecular-weight components after 12 months of storage at a temperature of 5°C ± 3°C under a closed condition is 0 to 1.00% as measured by SE-HPLC;
   **Content of Low-Molecular-Weight Components** (a peak whose retention time is later than that of the main peak (intact IgG)
- a liquid pharmaceutical formulation in which the content of low-molecular-weight components after 12 months of storage at a temperature of 5°C ± 3°C is 0 to 0.40% as measured by SE-HPLC;
- a liquid pharmaceutical formulation in which the content of low-molecular-weight components after 12 months of storage at a temperature of 5°C ± 3°C under a closed condition is 0 to 0.40% as measured by SE-HPLC;
   **Content of Intact Immunoglobulin G**
- a liquid pharmaceutical formulation in which the content of intact immunoglobulin G (intact IgG %) after 12 months of storage at a temperature of 5°C ± 3°C is 94.0% to 100% as measured by Non-reducing CE-SDS;
- a liquid pharmaceutical formulation in which the content of intact immunoglobulin G (intact IgG %) after 12 months of storage at a temperature of 5°C ± 3°C under a closed condition is 94.0% to 100% as measured by Non-reducing CE-SDS;
- a liquid pharmaceutical formulation in which the content of intact immunoglobulin G (intact IgG %) after 4 weeks of storage at a temperature of 40°C ± 2°C is 94.0% to 100% as measured by Non-reducing CE-SDS;
- a liquid pharmaceutical formulation in which the content of intact immunoglobulin G content (intact IgG %) after 4 weeks of storage at a temperature of 40°C ± 2°C and a relative humidity of 75 ± 5% under a closed condition is 94.0% to 100% as measured by Non-reducing CE-SDS;
   **Content of Intact Heavy Chain and Light Chain**
- a liquid pharmaceutical formulation in which the content of intact heavy chain and light chain (intact HC+LC %) after 12 months of storage at a temperature of 5°C ± 3°C is 99.0% to 100% as measured by reducing CE-SDS;
- a liquid pharmaceutical formulation in which the content of intact heavy chain and light chain (intact HC+LC %) after 12 months of storage at a temperature of 5°C ± 3°C under a closed condition is 99.0% to 100% as measured by reducing CE-SDS;

- a liquid pharmaceutical formulation in which the content of intact heavy chain and light chain (intact HC+LC %) after 4 weeks of storage at a temperature of 40°C ± 2°C is 98.0% to 100% as measured by reducing CE-SDS;
- a liquid pharmaceutical formulation in which the content of intact heavy chain and light chain content (intact HC+LC %) after 4 weeks of storage at a temperature of 40°C ± 2°C under a closed condition is 98.0% to 100% as measured by reducing CE-SDS;
   **Number of sub-visible particles**
- a liquid pharmaceutical formulation in which the number of sub-visible particles (≥10.00 µm, <400.00 µm) after 12 months of storage at a temperature of 5°C ± 3°C is 0 to 1,000 as measured by HIAC;
- a liquid pharmaceutical formulation in which the number of sub-visible particles (≥10.00 µm, <400.00 µm) after 12 months of storage at a temperature of 5°C ± 3°C under a closed condition is 0 to 1,000 as measured by HIAC;
- a liquid pharmaceutical formulation in which the number of sub-visible particles (≥1.00 µm, <100.00 µm) after 4 weeks of storage at a temperature of 40°C ± 2°C is 0 to 30,000 as measured by MFI;
- a liquid pharmaceutical formulation in which the number of sub-visible particles (≥1.00 µm, <100.00 µm) after 4 weeks of storage at a temperature of 40°C ± 2°C and a relative humidity of 75 ± 5% under a closed condition is 0 to 30,000 as measured by MFI;
- a liquid pharmaceutical formulation in which the number of sub-visible particles (≥10.00 µm, <100.00 µm) after 4 weeks of storage at a temperature of 40°C ± 2°C is 0 to 200 as measured by MFI;
- a liquid pharmaceutical formulation in which the number of sub-visible particles (≥10.00 µm, <100.00 µm) after 4 weeks of storage at a temperature of 40°C ± 2°C and a relative humidity of 75 ± 5% under a closed condition is 0 to 200 as measured by MFI;
- a liquid pharmaceutical formulation in which the number of sub-visible particles (≥10.00 µm, <100.00 µm) after 6 weeks of storage at a temperature of 40°C ± 2°C is 0 to 500 as measured by MFI;
- a liquid pharmaceutical formulation in which the number of sub-visible particles (≥10.00 µm, <100.00 µm) after 6 weeks of storage at a temperature of 40°C ± 2°C and a relative humidity of 75 ± 5% under a closed condition is 0 to 500 as measured by MFI;
   **Oxidation Rate**
- a liquid pharmaceutical formulation in which the oxidation rate of heavy-chain Met 255 after 4 weeks of storage at a temperature of 40°C ± 2°C is 0% to 2.5% as measured by LC-MS;
- a liquid pharmaceutical formulation in which the oxidation rate of heavy-chain Met 255 after 4 weeks of storage at a temperature of 40°C ± 2°C and a relative humidity of 75 ± 5% under a closed condition is 0% to 2.5% as measured by LC-MS;
   **Charge Variants**
- a liquid pharmaceutical formulation showing an acidic peak of 20% to 35% as measured by IEC-HPLC after 4 weeks of storage at a temperature of 40°C ± 2°C;
- a liquid pharmaceutical formulation showing an acidic peak of 20% to 35% as measured by IEC-HPLC after 4 weeks of storage at a temperature of 40°C ± 2°C and a relative humidity of 75 ± 5% under a closed condition;
- a liquid pharmaceutical formulation showing a basic peak of 33% to 40% as measured by IEC-HPLC after 4 weeks of storage at a temperature of 40°C ± 2°C;
- a liquid pharmaceutical formulation showing a basic peak of 33% to 40% as measured by IEC-HPLC after 4 weeks of storage at a temperature of 40°C ± 2°C and a relative humidity of 75 ± 5% under a closed condition;
   **TNF-α Binding Affinity**
- a liquid pharmaceutical formulation having a TNF-α binding affinity of 80% to 120% as measured by ELISA after 12 months of storage at a temperature of 5°C ± 3°C; and
- a liquid pharmaceutical formulation having a TNF-α binding affinity of 80% to 120% as measured by ELISA after 12 months of storage at a temperature of 5°C ± 3°C under a closed condition.

In one embodiment of the present invention, the pharmaceutical formulation may have a viscosity of 0.5 cp to 10.0 cp as measured after 1 month of storage at a temperature of 40°C ± 2°C. In another embodiment of the present invention, the pharmaceutical formulation may have a viscosity of 0.5 cp to 5.0 cp as measured after 6 months of storage at a temperature of 5°C ± 3°C.

### (H)Method for Preparation of Stable Liquid Pharmaceutical Formulation

The stable liquid pharmaceutical formulation of the present invention may be prepared using any known method which is not limited to a particular method. For example, the stable liquid pharmaceutical formulation may be prepared by adding a buffer to a solution containing a surfactant and a sugar or its derivative while adjusting the pH of the solution, and then adding an antibody to the mixed solution. Alternatively, the liquid pharmaceutical formulation may be prepared by preparing a solution containing some excipients in the final step of a purification process, and then adding the remaining component to the solution. For example, the liquid pharmaceutical formulation may be prepared by preparing a solution containing an antibody, a buffer and a sugar or its derivative, and then adding a surfactant to the solution.

In addition, the method for preparation of the formulation may comprise or not comprise a freeze-drying step.

When the preparation method does not comprise the freeze-drying step, for example, the liquid pharmaceutical formulation prepared according to the present invention may be treated by sterilization, and then immediately placed in a closed container.

When the preparation method comprises the freeze-drying step, for example, the liquid pharmaceutical formulation prepared according to the present invention may be freeze-dried or freeze-dried and stored, and then components removed or modified by freeze drying and/or storage may be supplemented or replaced, thereby preparing the liquid pharmaceutical formulation according to the present invention. Alternatively, only components of the liquid pharmaceutical formulation of the present invention, excluding components that may be removed or modified by freeze drying and/or storage, may be freeze-dried or freeze-dried and stored, and then the excluded components may be added thereto, thereby preparing the liquid pharmaceutical formulation according to the present invention.

Korean Patent Application No. 10-2017-0081814 previously filed by the Applicant is incorporated herein by reference.

### - Method for treatment of disease treatable with anti-TNF-α antibody of the present invention

The present invention provides a method for treatment of a disease treatable with anti-TNF-a, the method comprising a step of administering subcutaneously to a subject a pharmaceutical composition containing an anti-TNF-a antibody or its antigen binding fragment.

In one embodiment of the present invention, the antibody may comprise infliximab, adalimumab, certolizumab pegol, golimumab, or biosimilar thereof.

In one embodiment of the present invention, the antibody may comprise infliximab.

In one embodiment of the present invention, the antibody may comprise a chimeric human-mouse IgG monoclonal antibody.

In one embodiment of the present invention, the antibody or its the antigen binding fragment thereof may comprise: a light-chain variable region comprising a CDR1 domain comprising an amino acid sequence of SEQ ID NO: 1, a CDR2 domain comprising an amino acid sequence of SEQ ID NO: 2, and a CDR3 domain comprising an amino acid sequence of SEQ ID NO: 3; and a heavy-chain variable region comprising a CDR1 domain comprising an amino acid sequence of SEQ ID NO: 4, a CDR2 domain comprising an amino acid sequence of SEQ ID NO: 5, and a CDR3 domain comprising an amino acid sequence of SEQ ID NO: 6.

In one embodiment of the present invention, the antibody or its antigen binding fragment may comprise: a light-chain variable region having an amino acid sequence of SEQ ID NO: 7; and a heavy-chain variable region having an amino acid sequence of SEQ ID NO: 8.

In one embodiment of the present invention, the antibody may comprise: a light chain having an amino acid sequence of SEQ ID NO: 9; and a heavy chain having an amino acid sequence of SEQ ID NO: 10.

In one embodiment of the present invention, the antibody or its antigen binding fragment (A) may be contained at a concentration of 10 to 200 mg/ml.

The present invention also provides a method for treatment of a disease treatable with anti-TNF-a, the method comprising a step of administering subcutaneously to a subject a pharmaceutical composition containing (A) an anti-TNF-a antibody or its antigen binding fragment; (B) a surfactant; (C) a sugar or its derivative; (D) a buffer.

In one embodiment of the present invention, the surfactant (B) may comprise polysorbate, poloxamer, or a mixture thereof.

In one embodiment of the present invention, the surfactant (B) may comprise polysorbate 20, polysorbate 40, polysorbate 60, polysorbate 80, or a mixture of two or more thereof.

In one embodiment of the present invention, the surfactant (B) may comprise polysorbate 80.

In one embodiment of the present invention, the surfactant (B) may be contained at a concentration of 0.02 to 0.1% (w/v).

In one embodiment of the present invention, the sugar (C) may comprise a monosacchride, a disaccharide, an oligosaccharide, a polysaccharide, or a mixture of two or more thereof, and the sugar derivative (C) may comprise sugar alcohol, sugar acid, or a mixture thereof.

In one embodiment of the present invention, the sugar or its derivative (C) may comprise sorbitol, mannitol, trehalose, sucrose, or a mixture of two or more thereof.

In one embodiment of the present invention, the sugar or its derivative (C) may be contained at a concentration of 1 to 10% (w/v).

In one embodiment of the present invention, the buffer (D) may comprise acetate or histidine.

In one embodiment of the present invention, the buffer (D) may have a concentration of 1 to 50 mM.

In one embodiment of the present invention, the stable liquid pharmaceutical formulation may have a pH of 4.0 to 5.5.

In one embodiment of the present invention, the stable liquid pharmaceutical formulation may be free of aspartic acid, lysine, arginine, or mixtures thereof.

In one embodiment of the present invention, the stable liquid pharmaceutical formulation may be free of NaCl, KCl, NaF, KBr, NaBr, Na₂SO₄, NaSCN, K₂SO₄, or mixtures thereof.

In one embodiment of the present invention, the stable liquid pharmaceutical formulation may be free of a chelating agent.

In one embodiment of the present invention, the stable liquid pharmaceutical formulation may be free of a preservative.

In one embodiment of the present invention, the stable liquid pharmaceutical formulation may further contain an aqueous carrier, an antioxidant, or a mixture of two or more thereof.

In one embodiment of the present invention, the stable liquid pharmaceutical formulation may have a viscosity of 0.5 cp to 10 cp as measured after 1 month of storage at a temperature of 40°C ± 2°C, or a viscosity of 0.5 cp to 5 cp as measured after 6 months of storage at a temperature of 5°C ± 3 °C.

In one embodiment of the present invention, the stable liquid pharmaceutical formulation may contain: (A) an antibody or its antigen binding fragment, which comprises a light-chain variable region comprising a CDR1 domain comprising an amino acid sequence of SEQ ID NO: 1, a CDR2 domain comprising an amino acid sequence of SEQ ID NO: 2, and a CDR3 domain comprising an amino acid sequence of SEQ ID NO: 3; and a heavy-chain variable region comprising a CDR1 domain comprising an amino acid sequence of SEQ ID NO: 4, a CDR2 domain comprising an amino acid sequence of SEQ ID NO: 5, and a CDR3 domain comprising an amino acid sequence of SEQ ID NO: 6; (B) a surfactant; (C) a sugar or its derivative; and (D) a buffer comprising acetate or histidine.

In one embodiment of the present invention, the stable liquid pharmaceutical formulation may contain: (A) 90 to 180 mg/ml of an antibody or its antigen binding fragment, which comprises a light-chain variable region comprising a CDR1 domain comprising an amino acid sequence of SEQ ID NO: 1, a CDR2 domain comprising an amino acid sequence of SEQ ID NO: 2, and a CDR3 domain comprising an amino acid sequence of SEQ ID NO: 3; and a heavy-chain variable region comprising a CDR1 domain comprising an amino acid sequence of SEQ ID NO: 4, a CDR2 domain comprising an amino acid sequence of SEQ ID NO: 5, and a CDR3 domain comprising an amino acid sequence of SEQ ID NO: 6; (B) 0.02 to 0.1% (w/v) of a surfactant; (C) 1 to 10% (w/v) of a sugar or its derivative; and (D) 1 to 50 mM of a buffer comprising acetate or histidine.

In one embodiment of the present invention, the stable liquid pharmaceutical formulation may be administered subcutaneously.

In one embodiment of the present invention, the stable liquid pharmaceutical formulation may not be subjected to a reconstitution step, a dilution step, or both, before use.

In one embodiment of the present invention, the stable liquid pharmaceutical formulation may be filled in a pre-filled syringe before use.

In one embodiment of the present invention, the pre-filled syringe may be included in an auto-injector before use.

### Disease Treatable with Anti-TNF-a Antibody

In one embodiment of the present invention, the disease treatable with the anti-TNF-a antibody is selected from the group consisting of rheumatoid arthritis, ulcerative colitis, Crohn's disease, plaque psoriasis, psoriatic arthritis, ankylosing spondylitis, juvenile idiopathic arthritis, hemolytic disease of the newborn, inflammatory bowel disease, multiple sclerosis, prevention of organ transplantation rejection, non-Hodgkin's lymphoma, metastatic cancer, retinopathy of prematurity, ovarian cancer, stomach cancer, head and neck cancer, osteoporosis, paroxymal nocturnal hemoglobinuria, invasive candidiasis, breast cancer, melanoma, chronic lymphocytic leukemia, acute myeloid leukemia, renal cell carcinoma, colorectal cancer, asthma, nasopharyngeal cancer, hemorrhagic shock, Staphylococcus aureus infection, and follicular lymphoma.

In one embodiment of the present invention, the disease treatable with the anti-TNF-a antibody may be a disease treatable by intravenous administration of infliximab.

In one embodiment of the present invention, the disease treatable with the anti-TNF-a antibody may be rheumatoid arthritis, ulcerative colitis, Crohn's disease, plaque psoriasis, psoriatic arthritis or ankylosing spondylitis, which is treatable by intravenous administration of infliximab.

In one embodiment of the present invention, the subject to be administered with the anti-TNF-a antibody is a patient who has an inadequate response to disease-modifying anti rheumatic drugs (DMARDs), including methotrexate.

In one embodiment of the present invention, the subject to be administered with the anti-TNF-a antibody is a patient who has not previously been treated with methotrexate and other DMARDs.

In one embodiment of the present invention, the subject to be administered with the anti-TNF-a antibody is a patient who exhibits elevated serologic indicators associated with severe axial-predominant symptoms and inflammation, which show no proper response to common therapies.

In one embodiment of the present invention, the subject to be administered with the anti-TNF-a antibody is a patient who does not respond to, or are contraindicated to, or has intolerance to methotrexate, cyclosporine, or systemic therapies including psoralen ultraviolet A therapy (PUVA).

In one embodiment of the present invention, the subject to be administered with the anti-TNF-a antibody is a patient who has an inadequate response to, or has intolerance to, or is contraindicated for treatment with corticosteroids, 6-mercaptopurine, azathioprine or immunosuppressants.

In one embodiment of the present invention, the subject to be administered with the anti-TNF-a antibody is a patient who does not respond to common therapies, including antibiotic, excretion or immunosuppressive therapies.

### Dose and Administration Interval

In one embodiment of the present invention, the anti-TNF-α antibody or its antigen binding fragment may be administered at a dose of 60 to 300 mg. Specifically, it may be administered at a dose of 60, 70, 80, 90, 100, 110, 120, 130, 140, 150, 160, 170, 180, 190, 200, 210, 220, 230, 240, 250, 260, 270, 280, 290 or 300 mg.

In one embodiment of the present invention, the anti-TNF-α antibody or its antigen binding fragment may be administered at a dose of 90 to 180 mg. In another embodiment, the anti-TNF-α antibody or its antigen binding fragment may be administered at a dose of 120 to 240 mg. In still another embodiment, the anti-TNF-α antibody or its antigen binding fragment may be administered at a dose of 120 to 240 mg.

In one embodiment of the present invention, the anti-TNF-α antibody or its antigen binding fragment may be administered at a dose of 90 to 180 mg when the body weight of the patient is less than 80 kg, and may be administered in an amount of 190 to 270 mg when the body weight of the patient is more than 80 kg.

In one embodiment of the present invention, the anti-TNF-α antibody or its antigen binding fragment may be administered at intervals of 1 to 8 weeks. Specifically, it may be administered at intervals of 1 week, 1.5 weeks, 2 weeks, 2.5 weeks, 3 weeks, 3.5 weeks, 4 weeks, 4.5 weeks, 5 weeks, 5.5 weeks, 6 weeks, 6.5 weeks, 7 weeks, 7.5 weeks, or 8 weeks.

In another embodiment of the present invention, the anti-TNF-α antibody or its antigen binding fragment may be administered at intervals of 2 to 4 weeks.

### Pre-administration

Before the step of subcutaneously administering the anti-TNF-α antibody or its antigen binding fragment, a step of intravenously administering the anti-TNF-a antibody or its antigen binding fragment may be included.

In one embodiment of the present invention, before the subcutaneous administration step, a step of intravenously administering the anti-TNF-a antibody or its antigen binding fragment at a dose of 1 to 10 mg/kg may be included. Specifically, a step of intravenously administering the anti-TNF-α antibody or its antigen binding fragment at a dose of 1, 2, 3, 4, 5, 6, 7, 8, 9 or 10 mg/kg may be included.

In another embodiment of the present invention, before the subcutaneous administration step, a step of intravenously administering the anti-TNF-a antibody or its antigen binding fragment at a dose of 2 to 8 mg/kg may be included. In still another embodiment of the present invention, before the subcutaneous administration step, a step of intravenously administering the anti-TNF-a antibody or its antigen binding fragment at a dose of 3 to 5 mg/kg may be included.

In one embodiment of the present invention, before the subcutaneous administration step, a step of intravenously administering the anti-TNF-a antibody or its antigen binding fragment at intervals of 1 to 8 weeks may be included. Specifically, a step of intravenously administering the anti-TNF-α antibody or its antigen binding fragment at intervals of 1, 1.5, 2, 2.5, 3, 3.5, 4, 4.5, 5, 5.5, 6, 6.5, 7, 7.5 or 8 weeks may be included.

In another embodiment of the present invention, before the subcutaneous administration step, a step of intravenously administering the anti-TNF-a antibody or its antigen binding fragment at intervals of 2 to 4 weeks may be included.

In one embodiment of the present invention, before the subcutaneous administration step, a step of intravenously administering the anti-TNF-a antibody or its antigen binding fragment may be included, and the time interval between the last intravenous administration and the first subcutaneous administration is 1 to 8 weeks. Specifically, a step of intravenously administering the anti-TNF-a antibody or its antigen binding fragment at intervals of 1, 1.5, 2, 2.5, 3, 3.5, 4, 4.5, 5, 5.5, 6, 6.5, 7, 7.5 or 8 weeks may be included.

In another embodiment of the present invention, before the subcutaneous administration step, a step of intravenously administering the anti-TNF-a antibody or its antigen binding fragment may be included, and the time interval between the last intravenous administration and the first subcutaneous administration is 2 to 4 weeks.

### Co-administration

Other biological agent or chemotherapeutic agent may be administered together with the anti-TNF-a antibody or its antigen binding fragment of the present invention.

The administration is performed simultaneously with, before or after administration of the anti-TNF-a antibody or its antigen binding fragment.

In one embodiment of the present invention, the biological agent that is co-administered may comprise etanercept, infliximab, adalimumab, certolizumab pegol, golimumab, or a combination thereof.

In one embodiment of the present invention, the chemotherapeutic agent that is co-administered may comprise a disease-modifying anti rheumatic drug (DMARD).

In one embodiment of the present invention, the chemotherapeutic agent that is co-administered may comprise methotrexate, leflunomide, sulfasalazine, hydroxychloroquine, or a combination thereof.

### -Product

The present invention also provides a product comprising: the stable liquid pharmaceutical formulation; and a container receiving the stable liquid pharmaceutical formulation in a closed state.

The stable liquid pharmaceutical formulation is as described above.

In one embodiment of the present invention, the container may be formed of a material such as glass, a polymer (plastic), a metal or the like, but is not limited thereto. In one embodiment of the present invention, the container is a bottle, a vial, a cartridge, a syringe (pre-filled syringe, auto-syringe), or a tube, but is not limited thereto. In one embodiment of the present invention, the container may be a glass or polymer vial, or a glass or polymer pre-filled syringe.

Specific product forms of the above-described vial, cartridge, pre-filled syringe or auto-syringe, and methods of filling the stabile liquid pharmaceutical formulation into the vial, cartridge, pre-filled syringe or auto-syringe, may be readily available or implemented by any person skilled in the technical field to which the present invention pertains. For example, US Patent Nos. 4,861,335 and 6,331,174, etc., disclose the specific product form of a pre-filled syringe and a filling method. For example, US Patent Nos. 5,085,642 and 5,681,291, etc., disclose the specific product form of an auto-syringe and an assembly method. The above-described vial, cartridge, pre-filled syringe or auto-syringe that is used in the present invention may be a commercially available product, or a product separately manufactured considering the physical properties of the stable liquid pharmaceutical formulation, an area to which the formulation is to be administered, the dose of the formulation, and the like.

In one embodiment of the present invention, the inside of the container may not be coated with silicone oil. If it is coated with silicone oil, the stability of the formulation may be reduced. The container may be a single-dose or multiple-dose container.

In one embodiment of the present invention, the product may further comprise instructions providing a method of using the stable liquid pharmaceutical formulation, a method of storing the formulation, or both. The method of using the formulation includes a method for treating a disease in which TNF-α activity acts as a harmful factor, and may include the route of administration, the dose of the formulation, and the timing of administration.

In one embodiment of the present invention, the product may comprise other required utensils (e.g., a needle, a syringe, etc.) in a commercial viewpoint and a user viewpoint.

Hereinafter, the present invention will be described with reference to examples. It is to be understood, however, that these examples are for illustrative purposes only and are not intended to limit the scope of the present invention.

### Example 1. Evaluation of safety and therapeutic efficacy on subcutaneously administering an infliximab to patients with rheumatoid arthritis (RA)

The present clinical trial of an infliximab was a randomized, multi-center, parallel-group and phase I/III trial, designed to evaluate efficacy, pharmacokinetics and safety between the infliximab for subcutaneous administration (hereinafter infliximab SC) and the infliximab for intravenous administration (hereinafter infliximab IV) in combination with methotrexate (MTX) and folic acid in patients with active rheumatoid arthritis, who had not shown adequate responses to MTX administration over at least 3 months, wherein the present clinical trial was composed of two parts.

A part 1 was designed to find an optimal dose of the infliximab SC, wherein the optimal dose of the infliximab SC corresponding to 3 mg/kg of the infliximab IV over the first 30 weeks was identified by means of an area under the concentration-time curve (AUC_{τ}) at steady state between Weeks 22 and 30. In case of the part 1, a clinical trial period amounted to a maximum of 65 weeks, inclusive of duration from a screening (the maximum three weeks) to End-of-Study visit.

A part 2 was designed to demonstrate the non-inferiority of efficacy between the infliximab SC and the infliximab IV. Thus, by means of the clinical responses according to changes from baseline in disease activity score (DAS28; Disease Activity Score in 28 joints) (C-reactive protein, CRP) using 28 joint counts at Week 22, it might be demonstrated that the infliximab SC was not inferior to the infliximab IV in terms of efficacy. In case of the part 2, the administered dose and dosing interval of the infliximab SC were set to an administration of 120 mg every two weeks.

### Part 1

Patients were eligible to enroll into the present clinical trial, provided that they met all of the following criteria:
* Patient has active disease as defined by the presence of 6 or more swollen joints (of 28 assessed), 6 or more tender joints (of 28 assessed), and a serum CRP concentration >0.6 mg/dL at Screening; and
* Patient who has completed at least 3 months of treatment of oral or parenteral dosing with methotrexate between 12.5 to 25 mg/week and on stable dosing with methotrexate between 12.5 to 25 mg/week for at least 4 weeks prior to the first administration of the study drug (Day 0).

Patients were not eligible to enroll into the clinical trial, provided that they met any one of the following criteria:
* Patient who has previously received a biological agent for the treatment of RA and/or a TNF-α inhibitor for the treatment of other disease; and
* Patient who has allergies to any of the excipients of infliximab or any other murine and/or human proteins or patient with a hypersensitivity to immunoglobulin product.

The present clinical trial was composed of three clinical trial periods: Screening; Treatment period; and End-of-Study visit. The screening was carried out between Days -21 and -1 before an initial administration of the study drug, wherein the eligibility of patients for study was evaluated. All the examinations including hepatitis B, hepatitis C and human immunodeficiency virus (HIV-1 and HIV-2) infections, a urine and serum pregnancy test for women of childbearing potential, as well as rheumatoid factor, anti-cyclic citrullinated peptide, 12-lead ECG, clinical laboratory tests, etc., were carried out. Also, an interferon-gamma release assay (IGRA) and a chest X-ray examination were performed so as to exclude tuberculosis (TB) patients.

All the patients enrolled into the clinical trial were administered infliximab IV at Weeks 0 and 2, respectively. Further, the patients were administered folic acid in combination with the MTX and the study drug so that adverse events associated with the MTX side effects might be minimized and prevented, wherein they were also reminded of taking a maintenance dose of the MTX from beginning to end of clinical trial. Furthermore, the patients were eligible to take following premedication at an investigator's discretion at a time point of 30 - 60 minutes before a start of the administration of the study drug so that their hypersensitivity reactions to the study drug might be prevented: e.g., antihistamine (equivalent dose of 2 - 4 mg of chlorpheniramine), hydrocortisone, paracetamol and/or nonsedating antihistamine (equivalent dose of 10 mg of cetirizine), but not limited thereto.

Those who were administered a full dose of the study drug twice and deemed to have no concerns about safety at the investigator's discretion, were randomly assigned to an infliximab SC cohort or an infliximab IV cohort before the administration at Week 6/Day 42. Such random assignment with regard to administration of the study drug was stratified by country, a serum CRP concentration (same or less than 0.6 mg/dL or less, or more than 0.6 mg/dL) at Week 2 and a weight (same or less than 70 kg, or more than 70 kg) at Week 6. A total of 50 patients with active RA were enrolled, out of which 48 ones were randomly assigned to four clinical trial Cohorts at a ratio of 1:1:1:1, wherein the administration of the study drug was performed until Week 54 (Table 1).

**[Table 1]**

| **Cohort Number** | **Administered Dose** | **Drugs for Clinical Trials** | **Administration method** | **Number of Patients** |
|---|---|---|---|---|
| Cohort 1 | 3 mg/kg | Infliximab IV 100 mg/vial | 2 hours IV infusion | 13 |
| Cohort 2 | 90 mg | Infliximab SC 90 m/PFS | Single SC injection | 11 |
| Cohortt 3 | 120 mg | Infliximab SC 120 m/PFS | Single SC injection | 12 |
| Cohort 4 | 180 mg | Infliximab SC 90 m/PFS | double SC injection | 12 |

PFS, Pre-filled syringe

Those who were assigned to a cohort 1 were administered additional 7 doses of the infliximab IV at Week 6 and subsequently every eight weeks (Weeks 14, 22, 30, 38, 46 and 54). Those who were assigned to Cohorts 2, 3 and 4 were initially administered the infliximab SC at Week 6, and then additionally administered the infliximab SC every two weeks until Week 54. The initially assigned dose was adjusted to the optimal dose in all patients from Cohorts 2, 3 and 4 when the optimal dose was confirmed after dose finding. After that, an additional SC injection using the optimal dose was administered until Week 54. The infliximab SC was injected into patients by a healthcare professional at each visit (Weeks 6, 8, 10, 14, 22, 24, 26, 28, 30, 38, 46 and 54) at a study site. However, in all the other weeks (Weeks 12, 16, 18, 20, 32, 34, 36, 40, 42, 44, 48, 50 and 52), patients were allowed to perform a self-injection of the infliximab SC, if the investigator determined it as suitable after training them for appropriate injection techniques.

Patients visited the study site at a predefined time interval for clinical evaluation and blood sampling. At each visit, they were asked questions about adverse events and concomitant medication, while being monitored for clinical signs and symptoms of TB. The evaluation of primary pharmacokinetic endpoints was performed during a maintenance phase between Weeks 22 and 30, then the evaluation of secondary pharmacokinetic endpoints was performed during an treatment period until Week 54, and then blood sampling for analysis as well as the evaluation of efficacy, PD and safety were respectively performed at a point of time specified in an evaluation schedule.

An End-of-Study Visit occurred 8 weeks after the last dose was received, either at the end of the Maintenance Phase or earlier if the patient withdrew from the study. At a time point of eight weeks after the last administration to patients, every effort was made to complete all the End-of-Study evaluations.

### Part 2

A part 2 commenced based on a review by the Data Safety Monitoring Board with regard to PK modeling report data including PK, efficacy, PD and safety data, which were identified over the first 30 weeks in the part 1.

The part 2 was composed of three clinical trial periods including Screening; Treatment Period (Dose-Loading Phase and Maintenance Phase) with a double-blinded period during Maintenance Phase up to Week 30 followed by an open-label period of 24 weeks; and End-of-Study. The screening was carried out between Days -42 and Day 0 before an initial administration of the study drug, wherein the eligibility of patients for study was evaluated. All the examinations including hepatitis B, hepatitis C and human immunodeficiency virus (HIV-1 and HIV-2) infections, a urine and serum pregnancy test for women of childbearing potential, as well as rheumatoid factor, anti-cyclic citrullinated peptide, 12-lead ECG, clinical laboratory tests, etc., were carried out. Also, an interferon-gamma release assay (IGRA) and a chest X-ray examination were performed so as to exclude tuberculosis (TB) patients.

All the patients enrolled into the clinical trial were administered a single dose of the infliximab IV at Weeks 0 and 2, respectively. Further, the patients were administered folic acid in combination with the MTX and the study drug so that the adverse events associated with the MTX side effects might be minimized and prevented, wherein they were also reminded of taking a maintenance dose of the MTX from beginning to end of clinical trial. Furthermore, the patients were eligible to take following premedication at an investigator's discretion at a time point of 30 - 60 minutes before a start of the administration of the study drug so that their hypersensitivity reactions to the study drug might be prevented: e.g., antihistamine (equivalent dose of 2 - 4 mg of chlorpheniramine), hydrocortisone, paracetamol and/or nonsedating antihistamine (equivalent dose of 10 mg of cetirizine), but not limited thereto.

Those who were administered a full dose of the study drug twice and deemed to have no concerns about safety at the investigator's discretion, were randomly assigned to an infliximab SC which was administered by pre-filled syringe (PFS) with placebo IV or an infliximab IV with placebo SC (PFS) before the administration at Week 6/Day 42. Such random assignment with regard to administration of the study drug was stratified by country, a serum CRP concentration (same or less than 0.6 mg/dL, or more than 0.6 mg/dL) at Week 2 and a weight (same or less than 100 kg, or more than 100 kg) at Week 6. A minimum of 218 patients with active RA were randomly assigned to two clinical trial arms at a ratio of 1:1, wherein the administration of the study drug was performed until Week 54. A double dummy design was used to maintain blind until Week 30 (Table 2).

**[Table 2]**

| **Arm Number** | **Administered Dose** | **Drugs for Clinical Trials** | **Administration method** | **Number of Patients** |
|---|---|---|---|---|
| **Arm 1** | 3 mg/kg | Infliximab IV 100 mg/vial | 2 hours IV infusion | at least 109 |
| **Arm 2** | 120 mg | Infliximab SC 120 mg/PFS | Single SC injection | at least 109 |

PFS, Pre-filled syringe

Those who were assigned to a arm 1 were administered an additional 3 doses of the infliximab IV at Week 6 and subsequently every eight weeks (Weeks 14 and 22) until Week 22, while the placebo SC was administered at Week 6 and subsequently every two weeks until Week 28. After that, the infliximab IV was switched to the infliximab SC (PFS) at Week 30. Then, the infliximab SC (PFS) was administered until Week 54. Those who were assigned to a arm 2 were initially administered the infliximab SC (PFS) at Week 6 and subsequently every two weeks, which continued until Week 54, while the placebo IV was administered at Weeks 6, 14 and 22.

The infliximab SC (the placebo SC during a double-blind period) was injected into patients by a healthcare professional at each visit (Weeks 6, 14, 22, 24 - 28 (those who visited for PK evaluation), 30, 38, 46 and 54) at a study site. However, in all the other weeks (Weeks 8, 10, 12, 16, 18, 20, 24 - 28 (those who did not visit for PK evaluation), 32, 34, 36, 40, 42, 44, 48, 50 and 52), patients were allowed to perform a self-injection of the infliximab SC (the placebo SC during the double-blind period), if the investigator determined it as suitable after training them for appropriate injection techniques.

In a certain country, the infliximab SC was self-injected by an auto-injector (AI) every two weeks starting from Week 46. Evaluations by Self Injection Assessment Questionnaire (SIAQ) prior and after self-injection of Infliximab SC, self-injection assessment checklist, and a potential hazards checklist were performed so that the usability of the infliximab SC (AI) might be evaluated.

In case of the part 2, clinical evaluation, blood sampling and study visits for each type were performed in the same way as shown in the part 1 as well as at a point of time specified in an evaluation schedule.

### Results

### 1-1. Safety Evaluation

### Summary of Adverse Events

The safety evaluation was performed with regard to secondary endpoints in the part 1, i.e., immunogenicity, hypersensitivity monitoring (including delayed hypersensitivity monitoring), measurement of vital signs (including blood pressure, heart and respiration rates, and body temperature), weight, interferon-gamma release assay (IGRA), chest X-ray, hepatitis B, hepatitis C and human immunodeficiency virus (HIV-1, HIV-2) infectious states, opinions about physical examination, 12-lead ECG, adverse events (hereinafter AEs) (including serious adverse events (hereinafter SAEs)), adverse events of special interest (infusion-related reaction/hypersensitivity reaction/anaphylactic reaction [Administration-related reaction], delayed hypersensitivity reaction, injection site reaction, infection and malignancies), signs and symptoms of TB, clinical laboratory analysis, pregnancy test, prior and concomitant medication, local site pains using a 10 cm visual analogue scale (VAS), etc.

The cumulative safety data included adverse events (and serious adverse events) reported until End-of-Study visit regardless of their correlation with a clinical drug, wherein an overall summary of AEs reported after treatment during a maintenance phase (Weeks 6 to 54) was presented in Table 3. In general, 109 treatment-emergent AEs (hereinafter TEAEs) were reported in 33 patients (68.8%) - nine patients (69.2%) from the IV cohort (Cohort 1) and 24 patients (68.6%) from the SC total Cohorts (Cohorts 2, 3 and 4) respectively, thus indicating that the proportion of the SC total Cohorts was similar to that of the IV cohort. Also, the intensity of most TEAEs was shown as a grade 1 or 2, wherein among all the TEAEs, the AEs reported in total 23 patients (47.9%) were regarded to be related to the study drug.

Treatment-emergent SAEs (hereinafter TESAEs) were reported in total six patients (12.5%) - one patient (7.7%) from the IV cohort (Cohort 1) and five patients (14.3%) from the SC total Cohorts (Cohorts 2 and 4), respectively. Out of the SC Cohorts, the TESAEs were not reported in the cohort 3. The intensity of the TESAEs was shown as the grade 2 or 3, out of which patients regarded to be associated with the study drug were reported for two patients (16.7%) in the cohort 4. Also, the administration of the study drug was discontinued for one patient (9.1%) in the cohort 2 after the administration at Week 30 as well as two patients (16.7%) in the cohort 3 after the administration at Weeks 30 and 32 respectively, as the investigator determined them as critical medical events among all the TESAEs.

Out of the TEAEs classified as administration-related reactions (ARRs), the infusion-related reaction, hypersensitivity or anaphylactic reactions were reported in total four patients (8.3%) - one patient (7.7%) from the IV cohort (Cohort 1) and three patients (8.6%) from the SC total Cohorts (Cohorts 2 and 3). Out of the TEAEs classified as ARRs, the infusion-related reaction, hypersensitivity or anaphylactic reactions were not reported in the cohort 4 out of the SC Cohorts. As the infliximab ARRs was associated with an anti-drug antibody (hereinafter ADA) (Remsima SmPC 2017), the presence of the ADA was evaluated for patients having shown ARRs. Patients who had positive results for the ADA and a neutralizing antibody (hereinafter NAb) amounted to total two (each one for the Cohorts 1 and 2). One patient of the cohort 2 was administered the infliximab IV twice during a dose loading period, i.e., at Weeks 0 and 2, and then administered the infliximab SC total twice, i.e., at Weeks 6 and 8. The ARRs was reported at Weeks 6 and 8, and was identified as positive for the ADA and the NAb at Week 6 and the End-of-Study visit (performed in ten weeks after the visit at Week 8). The ARRs was reported in one patient of the cohort 1 at Week 38, and was identified as positive for the ADA and the NAb at Weeks 30 and 38 as well as at the End-of-Study visit (performed in ten weeks after the visit at Week 38).

The TEAEs classified as the injection site reaction were reported in total five patients (14.3%) in the SC total Cohorts (Cohorts 2, 3 and 4), wherein the intensity thereof was all shown as the grade 1 or 2. The TEAEs classified as the injection site reaction were not reported in the IV cohort (Cohort 1). The presence of the ADA was also evaluated for patients having shown the injection site reaction, and one (Cohort 2) of the five patients have positive results for the ADA and the NAb at a visit at Week 6, i.e., a day before the occurrence of the TEAEs classified as the injection site reaction.

Treatment-emergent AEs classified as infection were reported for 5 patients (38.5%) and 13 patients (37.1%) in the IV cohort (Cohort 1) and SC total Cohorts (Cohort 2-4), respectively.

TEAEs leading to discontinuation of study drug were reported for six patients (17.1%) in SC Cohorts (Cohorts 2, 3 and 4), wherein the reported AEs were antiphospholipid syndrome, injection site reaction, ARRs, pulmonary TB and latent TB. In the IV cohort, the TEAEs classified as infection were not reported.

No deaths were reported during the study.

**[Table 3]**

| | **Cohort 1 IV 3 mg/kg (N=13)** | **Cohort 2 SC 90 mg (N=11)** | **Cohort 3 SC 120 mg (N=12)** | **Cohort 4 SC 180 mg (N=12)** | **Total SC (N=35)** |
|---|---|---|---|---|---|
| Number of Patients with at Least one TEAE (%) | 9 (69.2) | 7 (63.6) | 8 (66.7) | 9 (75.0) | 24 (68.6) |
| Related | 4 (30.8) | 5 (45.5) | 7 (58.3) | 7 (58.3) | 19 (54.3) |
| Unrelated | 9 (69.2) | 5 (45.5) | 2 (16.7) | 4 (33.3) | 11 (31.4) |
| Number of Patients with at Least one TESAE (%) | 1 (7.7) | 2 (18.2) | 0 | 3 (25.0) | 5 (14.3) |
| Related | 0 | 0 | 0 | 2 (16.7) | 2 (5.7) |
| Unrelated | 1 (7.7) | 2 (18.2) | 0 | 1 (8.3) | 3 (8.6) |
| Number of Patients with at Least One TEAE cassified as administration-related reactions (%) | 1 (7.7) | 1 (9.1) | 2 (16.7) | 0 | 3 (8.6) |
| Number of Patients with at Least One | 0 | 2 (18.2) | 1 (8.3) | 2 (16.7) | 5 (14.3) |
| TEAE cassified as Injection Site Reaction (%) | | | | | |
| Number of Patients with at Least One TEAE cassified as infection (%) | 5 (38.5) | 4 (36.4) | 3 (25.0) | 6 (50.0) | 13 (37.1) |
| Related | 2 (15.4) | 1 (9.1) | 3 (25.0) | 3 (25.0) | 7 (20.0) |
| Number of Patients with at Least One TEAE Leading to Study Drug Discontinuation (%) | 0 | 2 (18.2) | 2 (16.7) | 2 (16.7) | 6 (17.1) |

| | | | | | |
|---|---|---|---|---|---|
| * At each level of summarization, patients were counted once if they reported one or more events. Only the most severe event was counted. The event was considered to be related if the relationship was defined as 'Possible', 'Probable', 'Definite'. | | | | | |

### Immunogenicity

In general, the proportion of patients with positive ADA results was lower in the SC Cohorts during the study and the majority of patients had negative ADA test results at Week 54. Also there was a trend towards lower rates of ADA positive with increased Infliximab SC dosage. The number of patients who had positive ADA results at Week 54 was 9 (69.2%), 4 (36.4%), 2 (16.7%) and 2 (16.7%) patients from Cohort 1 to 4, respectively (Table 4).

**[Table 4]**

| **Visit** | **Cohort 1 IV 3 mg/kg (N=13)** | **Cohort 2 SC 90 mg (N=11)** | **Cohort 3 SC 120 mg (N=12)** | **Cohort 4 SC 180 mg (N=12)** | **Total SC (N=35)** |
|---|---|---|---|---|---|
| Week 0 | | | | | |
| ADA positive | 0 | 0 | 0 | 0 | 0 |
| NAb positive | 0 | 0 | 0 | 0 | 0 |

| Week 6 | | | | | |
|---|---|---|---|---|---|
| ADA positive | 0 | 2 (18.2) | 0 | 0 | 2 (5.7) |
| NAb positive | 0 | 2 (18.2) | 0 | 0 | 2 (5.7) |

| Week 14 | | | | | |
|---|---|---|---|---|---|
| ADA positive | 5 (38.5) | 2 (18.2) | 1 (8.3) | 1 (8.3) | 4 (11.4) |
| NAb positive | 3 (23.1) | 1 (9.1) | 1 (8.3) | 0 | 2 (5.7) |

| Week 22 | | | | | |
|---|---|---|---|---|---|
| ADA positive | 7 (53.8) | 3 (27.3) | 2 (16.7) | 1 (8.3) | 6 (17.1) |
| NAb positive | 5 (38.5) | 2 (18.2) | 1 (8.3) | 0 | 3 (8.6) |

| Week 30 | | | | | |
|---|---|---|---|---|---|
| ADA positive | 9 (69.2) | 4 (36.4) | 3 (25.0) | 0 | 7 (20.0) |
| NAb positive | 6 (46.2) | 2 (18.2) | 1 (8.3) | 0 | 3 (8.6) |

| Week 38 | | | | | |
|---|---|---|---|---|---|
| ADA positive | 9 (69.2) | 4 (36.4) | 1 (8.3) | 1 (8.3) | 6 (17.1) |
| NAb positive | 6 (46.2) | 3 (27.3) | 1 (8.3) | 0 | 4 (11.4) |

| Week 46 | | | | | |
|---|---|---|---|---|---|
| ADA positive | 7 (53.8) | 5 (45.5) | 2 (16.7) | 0 | 7 (20.0) |
| NAb positive | 5 (38.5) | 2 (18.2) | 1 (8.3) | 0 | 3 (8.6) |

| Week 54 | | | | | |
|---|---|---|---|---|---|
| ADA positive | 9 (69.2) | 4 (36.4) | 2 (16.7) | 2 (16.7) | 8 (22.9) |
| NAb positive | 5 (38.5) | 2 (18.2) | 1 (8.3) | 1 (8.3) | 4 (11.4) |
| at least one ADA positive" | 11/13 (84.6) | 5/9 (55.6) | 4/12 (33.3) | 3/12 (25.0) | 12/33 (36.4) |
| at least one NAb positive** | 7/13 (53.8) | 2/9 (22.2) | 1/12 (8.3) | 1/12 (8.3) | 4/33 (12.1) |

| | | | | | |
|---|---|---|---|---|---|
| * ADA, Anti-drug antibody; NAb, Neutralizing antibody ** Among the patients who had never been identified as positive for the ADA and the NAb before the study drug administration at Week 6, only the patients who had ever been identified as positive for the ADA and the NAb even once after the study drug administration at Week 6 were used for the counting. | | | | | |

### Local Site Pain Assessment Using the Visual Analogue Scale

The VAS range was from 0 to 100 mm, with higher scores indicating more severe pain. Slightly higher level of local site pain (VAS) was observed in SC Cohorts (Cohorts 2, 3, 4) compared to IV cohort (Cohort 1) at the time of first SC injection (at Week 6), however, local site pain gradually decreased as Infliximab SC was injected repeatedly, and lower level of the pain was reported for SC Cohorts (Cohorts 2, 3, 4) compared to IV cohort (Cohort 1). Among the SC Cohorts, the mean local site pain using the VAS of SC 180 mg cohort was slightly higher than other SC cohorts due to receiving double SC injection at one time, but there was a trend that the level of the pain in SC 180 mg cohort was lower than that of IV cohort (Cohort 1) (Table 5).

**[Table 5]**

| **Visit Statistic** | **Cohort 1 IV 3 mg/kg (N=13)** | **Cohort 2 SC 90 mg (N=11)** | **Cohort 3 SC 120 mg (N=12)** | **Cohort 4 SC 180 mg (N=12)** |
|---|---|---|---|---|
| Week 6 | | | | |
| N | 8 | 11 | 12 | 12 |
| Mean (Standard Deviation, SD) | 9.63 (12.817) | 7.09 (9.648) | 17.71 (21.922) | 11.21 (12.496) |
| Median | 5.00 | 2.00 | 8.00 | 7.50 |
| Minimum, Maximum | 1,39 | 0,30 | 0,65 | 0,35 |

| Week 14 | | | | |
|---|---|---|---|---|
| N | 10 | 10 | 11 | 12 |
| Mean (SD) | 11.70 (14.080) | 4.20 (6.210) | 7.82 (8.072) | 10.21 (13.934) |
| Median | 7.00 | 1.25 | 6.00 | 5.00 |
| Minimum, Maximum | 1,45 | 0, 18 | 0, 23 | 0,41 |

| Week 22 | | | | |
|---|---|---|---|---|
| N | 13 | 0 | 11 | 12 |
| Mean (SD) | 19.15 (32.080) | 5.30 (5.912) | 6.86 (10.460) | 10.17 (10.100) |
| Median | 3.00 | 2.25 | 2.00 | 7.00 |
| Minimum, Maximum | 0,84 | 0,18 | 0,36 | 1,31 |

| Week 30 | | | | |
|---|---|---|---|---|
| N | 13 | 0 | 11 | 12 |
| Mean (SD) | 13.85 (22.649) | 7.55 (10.828) | 4.36 (5.446) | 6.08 (5.351) |
| Median | 3.00 | 2.25 | 3.00 | 3.50 |
| Minimum, Maximum | 0,77 | 0, 35 | 0,17 | 1,16 |

| Week 54 | | | | |
|---|---|---|---|---|
| N | 12 | 9 | 9 | 8 |
| Mean (SD) | 5.58 (7.868) | 5.22 (5.974) | 5.22 (5.094) | 9.63 (6.718) |
| Median | 2.00 | 2.00 | 3.00 | 9.50 |
| Minimum, Maximum | 1,26 | 0,15 | 0,17 | 1,19 |

### 1-2. Therapeutic Efficacy Evaluation

### Disease activity measured by DAS28

As a result of analyzing DAS28(C reactive protein; CRP) and DAS28(erythrocyte sedimentation rate; ESR) at Weeks 2, 6, 14, 22, 30 and 54 in comparison with baseline as a secondary efficacy endpoint, there was no notable differences between the infliximab SC Cohorts with three different doses and the infliximab IV cohort, wherein it was also identified that actual values tended to decrease as time went by.

Actual values and changes from baseline in disease activity measured by DAS28 are summarized in Table 6 (CRP) and Table 7 (ESR). The mean scores for disease activity measured by DAS28 decreased from Baseline at Weeks 2, 6, 14, 22, 30 and 54 in each cohort. There was a trend towards slightly lower than IV cohort DAS28 (CRP) score in the SC Cohorts from Week 22 and DAS28 (ESR) score in the SC Cohorts from Week 30, which was consistent with the higher C_{trough} concentrations in the SC Cohorts.

**[Table 6]**

| | **IV 3mg/kg (N=13)** | | **SC 90mg (N=11)** | | **SC 120mg (N=12)** | | **SC 180mg (N=12)** | |
|---|---|---|---|---|---|---|---|---|
| **Visit Statistic** | **Actual Result** | **Change from Baseline** | **Actual Result** | **Change from Baseline** | **Visit Statistic** | **Actual Result** | **Change from Baseline** | **Actual Result** |
| Baseline | | | | | | | | |
| n | 13 | | 11 | | 12 | | 12 | |
| Mean | 5.413 | | 6.273 | | 5.719 | | 5.527 | |
| SD | 0.7776 | | 0.8234 | | 0.9120 | | 0.8185 | |
| Minimum | 4.11 | | 5.13 | | 4.53 | | 4.62 | |
| Median | 5.355 | | 6.265 | | 5.492 | | 5.299 | |
| Maximum | 6.68 | | 7.46 | | 7.31 | | 7.10 | |

| Week 2 | | | | | | | | |
|---|---|---|---|---|---|---|---|---|
| n | 13 | 13 | 11 | 11 | 12 | 12 | 12 | 12 |
| Mean | 4.328 | -1.086 | 5.030 | -1.243 | 4.421 | -1.297 | 4.230 | -1.297 |
| SD | 1.5161 | 0.9000 | 1.3922 | 1.2509 | 0.8815 | 0.5899 | 1.2647 | 0.8990 |
| Minimum | 1.71 | -2.41 | 2.27 | -4.09 | 3.39 | -2.10 | 2.60 | -2.94 |
| Median | 4.661 | -1.143 | 5.367 | -1.191 | 4.067 | -1.206 | 4.317 | -1.206 |
| Maximum | 6.59 | 0.34 | 6.35 | 0.24 | 6.48 | -0.23 | 6.59 | -0.04 |

| Week 6 | | | | | | | | |
|---|---|---|---|---|---|---|---|---|
| n | 13 | 13 | 11 | 11 | 12 | 12 | 11 | 11 |
| Mean | 3.902 | -1.512 | 4.600 | -1.673 | 3.921 | -1.798 | 3.419 | -2.007 |
| SD | 1.4765 | 0.9278 | 1.0735 | 0.7104 | 1.1911 | 0.8442 | 1.1775 | 0.9631 |
| Minimum | 1.82 | -2.75 | 2.90 | -2.37 | 2.55 | -3.25 | 1.56 | -3.97 |
| Median | 4.414 | -1.451 | 4.663 | -1.919 | 3.323 | -1.890 | 3.370 | -2.004 |
| Maximum | 6.37 | -0.28 | 6.24 | -0.03 | 6.28 | -0.43 | 5.09 | -0.27 |

| Week 14 | | | | | | | | |
|---|---|---|---|---|---|---|---|---|
| n | 13 | 13 | 10 | 10 | 11 | 11 | 12 | 12 |
| Mean | 3.558 | -1.856 | 3.968 | -2.296 | 3.446 | -2.255 | 2.933 | -2.594 |
| SD | 1.2612 | 0.8621 | 1.2485 | 0.8131 | 1.3406 | 1.1376 | 1.1779 | 0.8486 |
| Minimum | 1.92 | -2.89 | 1.84 | -3.43 | 1.39 | -4.41 | 1.50 | 4.00 |
| Median | 3.549 | -2.102 | 3.740 | -2.392 | 3.223 | -1.881 | 2.702 | -2.665 |
| Maximum | 6.15 | -0.52 | 5.58 | -1.18 | 5.83 | -0.89 | 5.63 | -1.00 |

| Week 22 | | | | | | | | |
|---|---|---|---|---|---|---|---|---|
| n | 13 | 13 | 10 | 10 | 11 | 11 | 12 | 12 |
| Mean | 3.885 | -1.529 | 3.730 | -2.534 | 3.257 | -2.444 | 2.789 | -2.738 |
| SD | 1.6167 | 1.3228 | 1.0430 | 0.8491 | 1.4077 | 1.2380 | 1.3266 | 0.8753 |
| Minimum | 1.86 | -3.33 | 1.69 | -4.07 | 1.50 | -4.35 | 1.11 | -3.95 |
| Median | 4.066 | -1.404 | 3.626 | -2.324 | 2.977 | -1.989 | 2.606 | -2.922 |
| Maximum | 6.74 | 1.38 | 5.27 | -1.45 | 5.79 | -1.07 | 5.53 | -1.10 |

| Week 30 | | | | | | | | |
|---|---|---|---|---|---|---|---|---|
| n | 13 | 13 | 10 | 10 | 10 | 10 | 12 | 12 |
| Mean | 3.315 | -2.099 | 3.029 | -3.236 | 3.105 | -2.586 | 2.741 | -2.786 |
| SD | 1.2515 | 0.8499 | 1.1446 | 1.2430 | 1.0076 | 0.9749 | 0.9661 | 0.5621 |
| Minimum | 1.80 | -3.02 | 1.35 | -6.11 | 1.77 | -4.56 | 1.15 | -3.79 |
| Median | 3.150 | -2.312 | 2.917 | -2.931 | 3.038 | -2.345 | 2.831 | -2.668 |
| Maximum | 6.41 | 0.26 | 4.78 | -1.81 | 4.74 | -1.18 | 4.51 | -2.04 |

| Week 54 | | | | | | | | |
|---|---|---|---|---|---|---|---|---|
| n | 12 | 12 | 8 | 8 | 10 | 10 | 10 | 10 |
| Mean | 3.548 | -1.961 | 3.025 | -3.090 | 3.167 | -2.545 | 2.911 | -2.505 |
| SD | 1.1928 | 1.0766 | 1.0242 | 0.7390 | 0.9706 | 1.3996 | 0.9929 | 0.9279 |
| Minimum | 1.26 | -3.60 | 1.57 | 4.44 | 1.35 | -4.46 | 1.11 | -3.51 |
| Median | 3.685 | -1.742 | 2.770 | -3.025 | 3.463 | -2.585 | 2.877 | -2.866 |
| Maximum | 5.28 | 0.08 | 4.58 | -1.93 | 4.60 | 0.00 | 4.31 | -0.55 |

**[Table 7]**

| | **IV 3mg/kg (N=13)** | | **SC 90mg (N=11)** | | **SC 120mg (N=12)** | | SC **180mg (N=12)** | |
|---|---|---|---|---|---|---|---|---|
| **Visit Statistic** | **Actual Result** | **Change from Baseline** | **Actual Result** | **Change from Baseline** | **Visit Statistic** | **Actual Result** | **Change from Baseline** | **Actual Result** |
| Baseline | | | | | | | | |
| n | 13 | | 11 | | 12 | | 12 | |
| Mean | 6.031 | | 7.091 | | 6.410 | | 6.362 | |
| SD | 0.8940 | | 0.8481 | | 1.1642 | | 0.8497 | |
| Minimum | 3.85 | | 5.33 | | 4.54 | | 5.04 | |
| Median | 6.009 | | 7.182 | | 6.360 | | 6.318 | |
| Maximum | 7.41 | | 8.18 | | 8.11 | | 7.98 | |

| Week 2 | | | | | | | | |
|---|---|---|---|---|---|---|---|---|
| n | 13 | 13 | 11 | 11 | 12 | 12 | 12 | 12 |
| Mean | 4.970 | -1.061 | 5.773 | -1.317 | 5.203 | -1.207 | 5.019 | -1.343 |
| SD | 1.7649 | 0.9720 | 1.8342 | 1.4313 | 1.3003 | 0.8837 | 1.3065 | 0.9234 |
| Minimum | 1.31 | -2.55 | 1.93 | -4.86 | 3.22 | -3.35 | 3.43 | -3.19 |
| Median | 5.481 | -0.816 | 6.433 | -1.116 | 5.073 | -1.014 | 4.793 | -1.285 |
| Maximum | 7.51 | 0.44 | 7.51 | 0.10 | 8.04 | -0.07 | 7.61 | -0.01 |

| Week 6 | | | | | | | | |
|---|---|---|---|---|---|---|---|---|
| n | 13 | 13 | 11 | 11 | 12 | 12 | 12 | 12 |
| Mean | 4.496 | -1.535 | 5.387 | -1.704 | 4.685 | -1.725 | 4.418 | -1.944 |
| SD | 1.5015 | 0.8285 | 1.1940 | 0.6497 | 1.3766 | 1.0068 | 1.4161 | 1.0233 |
| Minimum | 2.13 | -2.78 | 2.88 | -2.45 | 3.27 | -3.37 | 2.25 | -4.38 |
| Median | 4.714 | -1.151 | 5.331 | -1.775 | 4.184 | -1.551 | 4.361 | -1.919 |
| Maximum | 7.35 | -0.06 | 7.09 | -0.35 | 7.66 | -0.03 | 7.20 | -0.46 |

| Week 14 | | | | | | | | |
|---|---|---|---|---|---|---|---|---|
| n | 13 | 13 | 10 | 10 | 11 | 11 | 12 | 12 |
| Mean | 4.236 | -1.795 | 4.830 | -2.291 | 4.306 | -2.081 | 3.740 | -2.621 |
| SD | 1.2772 | 0.7825 | 1.4762 | 0.9005 | 1.3940 | 1.3178 | 1.1732 | 0.7395 |
| Minimum | 2.32 | -3.14 | 1.77 | -3.56 | 2.68 | -4.16 | 2.14 | -3.94 |
| Median | 4.065 | -1.588 | 4.821 | -2.288 | 4.220 | -1.395 | 3.417 | -2.732 |
| Maximum | 6.88 | -0.53 | 6.48 | -0.97 | 7.00 | -0.32 | 6.48 | -1.19 |

| Week 22 | | | | | | | | |
|---|---|---|---|---|---|---|---|---|
| n | 13 | 13 | 10 | 10 | 11 | 11 | 12 | 12 |
| Mean | 4.354 | -1.677 | 4.515 | -2.606 | 3.875 | -2.512 | 3.380 | -2.981 |
| SD | 1.8065 | 1.4558 | 1.2611 | 0.8066 | 1.8028 | 1.7593 | 1.1641 | 0.6857 |
| Minimum | 1.60 | -4.02 | 1.57 | -3.76 | 0.60 | -6.28 | 1.85 | -3.96 |
| Median | 4.267 | -1.479 | 4.483 | -2.470 | 3.890 | -1.706 | 2.989 | -2.908 |
| Maximum | 7.75 | 1.76 | 5.90 | -1.59 | 6.67 | -0.57 | 5.97 | -1.70 |

| Week 30 | | | | | | | | |
|---|---|---|---|---|---|---|---|---|
| n | 13 | 13 | 10 | 10 | 10 | 10 | 12 | 12 |
| Mean | 3.866 | -2.165 | 3.669 | -3.452 | 3.776 | -2.562 | 3.318 | -3.044 |
| SD | 1.3425 | 0.9643 | 1.3555 | 1.1242 | 1.1186 | 1.2496 | 1.0164 | 0.5380 |
| Minimum | 1.83 | -3.79 | 1.15 | -5.65 | 2.02 | -4.63 | 1.54 | -3.79 |
| Median | 4.156 | -2.226 | 3.517 | -3.239 | 3.642 | -2.401 | 3.088 | -2.992 |
| Maximum | 6.72 | -0.03 | 5.32 | -1.81 | 5.55 | -0.69 | 5.38 | -1.98 |

| Week 54 | | | | | | | | |
|---|---|---|---|---|---|---|---|---|
| n | 12 | 12 | 9 | 9 | 11 | 11 | 10 | 10 |
| Mean | 4.134 | -2.078 | 3.984 | -3.129 | 4.067 | -2.320 | 3.434 | -2.847 |
| SD | 1.3522 | 1.2491 | 1.1889 | 0.7665 | 0.9426 | 1.7582 | 1.1128 | 0.8623 |
| Minimum | 1.92 | -3.67 | 1.55 | -4.68 | 2.24 | -4.34 | 1.13 | -3.91 |
| Median | 4.112 | -2.001 | 4.305 | -3.117 | 4.337 | -2.602 | 3.284 | -2.970 |
| Maximum | 6.50 | 0.38 | 5.47 | -2.00 | 5.45 | 0.91 | 4.87 | -1.16 |

### The EULAR responses

The proportion of patients with a good or moderate response, defined according to the EULAR (European League Against Rheumatism) response criteria using DAS28 (CRP) during the study was similar among Cohorts (Table 8). The results of EULAR response criteria using DAS28 (ESR) response were also similar among Cohorts during the study (Table 9).

**[Table 8]**

| **Visit Statistic** | **IV 3 mg/kg (N=13)** | **SC 90 mg (N=11)** | **SC 120 mg (N=12)** | **SC 180 mg (N=12)** |
|---|---|---|---|---|
| Week 2 | | | | |
| Number of patients responded (%) | 7 (53.8) | 6 (54.5) | 10 (83.3) | 8 (66.7) |
| - Moderate Response | 3 (23.1) | 4 (36.4) | 10 (83.3) | 4 (33.3) |
| - Good Response | 4 (30.8) | 2 (18.2) | 0 | 4 (33.3) |

| Week 6 | | | | |
|---|---|---|---|---|
| Number of patients responded (%) | 9 (69.2) | 9 (81.8) | 10 (83.3) | 10 (88.3) |
| - Moderate Response | 4 (30.8) | 8 (72.7) | 5 (41.7) | 6 (50.0) |
| - Good Response | 5 (38.5) | 1 (9.1) | 5 (41.7) | 4 (33.3) |

| Week 14 | | | | |
|---|---|---|---|---|
| Number of patients responded (%) | 12 (92.3) | 9 (81.8) | 10 (83.3) | 11 (91.7) |
| - Moderate Response | 6 (46.2) | 7 (63.6) | 5 (41.7) | 2 (16.7) |
| - Good Response | 6 (46.2) | 2 (18.2) | 5 (41.7) | 9 (75.0) |

| Week 22 | | | | |
|---|---|---|---|---|
| Number of patients responded (%) | 10 (76.9) | 10 (90.9) | 10 (83.3) | 11 (91.7) |
| - Moderate Response | 5 (38.5) | 8 (72.7) | 3 (25.0) | 4 (33.3) |
| - Good Response | 5 (38.5) | 2 (18.2) | 7 (58.3) | 7 (58.3) |

| Week 30 | | | | |
|---|---|---|---|---|
| Number of patients responded (%) | 12 (92.3) | 10 (90.9) | 10 (83.3) | 12 (100.0) |
| - Moderate Response | 5 (38.5) | 5 (45.5) | 5 (41.7) | 5 (41.7) |
| - Good Response | 7 (53.8) | 5 (45.5) | 5 (41.7) | 7 (58.3) |

| Week 54 | | | | |
|---|---|---|---|---|
| Number of patients responded (%) | 11 (84.6) | 8 (72.7) | 9 (75.0) | 9 (75.0) |
| - Moderate Response | 7 (53.8) | 3 (27.3) | 6 (50.0) | 3 (25.0) |
| - Good Response | 4 (30.8) | 5 (45.5) | 3 (25.0) | 6 (50.0) |

**[Table 9]**

| **Visit Statistic** | **IV 3 mg/kg (N=13)** | **SC 90 mg (N=11)** | **SC 120 mg (N=12)** | **SC 180 mg (N=12)** |
|---|---|---|---|---|
| Week 2 | | | | |
| Number of patients responded (%) | 6 (46.2) | 5 (45.5) | 7 (58.3) | 9 (75.0) |
| - Moderate Response | 4 (30.8) | 3 (27.3) | 7 (58.3) | 9 (75.0) |
| - Good Response | 2 (15.4) | 2 (18.2) | 0 | 0 |

| Week 6 | | | | |
|---|---|---|---|---|
| Number of patients responded (%) | 8 (61.5) | 9 (81.8) | 10 (83.3) | 10 (83.3) |
| - Moderate Response | 5 (38.5) | 8 (72.7) | 10 (83.3) | 8 (66.7) |
| - Good Response | 3 (23.1) | 1 (9.1) | 0 | 2 (16.7) |

| Week 14 | | | | |
|---|---|---|---|---|
| Number of patients responded (%) | 11 (84.6) | 8 (82.7) | 9 (75.0) | 11 (91.7) |
| - Moderate Response | 8 (61.5) | 7(63.6) | 6 (50.0) | 6 (50.0) |
| - Good Response | 3 (23.1) | 1 (9.1) | 3 (25.0) | 5 (41.7) |

| Week 22 | | | | |
|---|---|---|---|---|
| Number of patients responded (%) | 10 (76.9) | 10 (90.9) | 10 (83.3) | 12 (100.0) |
| - Moderate Response | 6 (46.2) | 9 (81.8) | 6 (50.0) | 6 (50.0) |
| - Good Response | 4 (30.8) | 1 (9.1) | 4 (33.3) | 6 (50.0) |

| Week 30 | | | | |
|---|---|---|---|---|
| Number of patients responded (%) | 12 (92.3) | 10 (90.9) | 10 (83.3) | 12 (100.0) |
| - Moderate Response | 7 (53.8) | 6 (54.5) | 8 (66.7) | 4 (33.3) |
| - Good Response | 5 (38.5) | 4 (36.4) | 2 (16.7) | 8 (66.7) |

| Week 54 | | | | |
|---|---|---|---|---|
| Number of patients responded (%) | 11 (84.6) | 9 (81.8) | 9 (75.0) | 9 (75.0) |
| - Moderate Response | 9 (69.2) | 8 (72.7) | 7 (58.3) | 5 (41.7) |
| - Good Response | 2 (15.4) | 1 (9.1) | 2 (16.7) | 5 (41.7) |

### Proportion of Patients Achieving Clinical Response according to the ACR20

The proportion of patients achieving clinical response according to the ACR20 (American College of Rheumatology 20% improvement) criteria from Week 14 to Week 54 were similar between IV cohort (Cohort 1) and SC Cohorts (Cohort 2, 3, 4) (Table 10).

**[Table 10]**

| **Parameter Visit** | **Cohort 1 IV 3 mg/kg (N=13)** | **Cohort 2 SC 90 mg (N=11)** | **Cohort 3 SC 120 mg (N=12)** | **Cohort 4 SC 180 mg (N=12)** |
|---|---|---|---|---|
| ACR20 | | | | |
| Week 2 | 4 (30.8) | 3 (27.3) | 4 (33.3) | 6 (50.0) |
| Week 6 | 8 (61.5) | 8 (72.7) | 7 (58.3) | 7 (58.3) |
| Week 14 | 10 (76.9) | 8 (72.7) | 8 (66.7) | 11 (91.7) |
| Week 22 | 8 (61.5) | 8 (72.7) | 9 (75.0) | 11 (91.7) |
| Week 30 | 11(84.6) | 10 (90.9) | 10 (83.3) | 12 (100.0) |
| Week 54 | 9 (69.2) | 9 (81.8) | 8 (66.7) | 10 (83.3) |

### Example 2. Modeling for subcutaneously administering an infliximab to RA patients

### PK-PD Model Construction

A pharmacokinetic-pharmacodynamic (PK-PD) model for the infliximab subcutaneous (SC) administration was established to integrate with a quantitative pharmacokinetic (PK) model not only for simulating the PK of future administered doses and regimens, but also for simulating the efficacy and safety of the infliximab SC. The PK-PD model was based on the infliximab IV administration data on healthy volunteers, ankylosing spondylitis (AS) patients, rheumatoid arthritis (RA) patients and Crohn's disease (CD) patients, as well as the infliximab SC administration data on CD patients, RA patients and healthy volunteers (Clinicaltrials.gov Identifier Code NCT01220518, NCT01217086, NCT02096861).

The PK-PD model constructed based on the said data may be used to simulate the subcutaneous administration results for patients having the infliximab indications (RA, ulcerative colitis (UC), CD, plaque psoriasis, psoriatic arthritis or ankylosing spondylitis).

The PK-PD modeling analysis was performed by a nonlinear mixed effect modeling approach. The data analysis commenced by a 1-compartment model including a proportional elimination of a proportional error model, and then a final model was performed by a 2-compartment model having a linear elimination from a central compartment. All the models on pharmacokinetics were variablized in terms of clearance (CL) and volume of distribution.

As for the final PK model, a profile was predicted in such a way that each estimated value for area under the concentration-time curve (AUCₜₐᵤ) and minimum concentration immediately before the next study drug administration (C_{trough}) parameters with regard to the infliximab clinical trial was applied to each actual dose, usage and administration route, wherein the said data were descriptively summarized with a median value and a confidence interval of 90%. Also, an additional simulation was performed so as to evaluate an effect on a fixed dose, usage and administration route for each weight. The PK-PD modeling and simulation of subcutaneous doses were performed by means of NONMEM v7.2.

### Dose-regimen scenario of SC dosage form for modeling in RA patients

A pharmacokinetic aspect was predicted based on a following simulation scenario, wherein accordingly efficacy and safety were predicted, and then evaluated in comparison with an infliximab IV infusion usage, in which a maintenance dose of 3 mg/kg was administered at an interval of eight weeks (Table 11) :

**[Table 11]**

| |
|---|
| Infliximab SC 120 mg Every 2 weeks |
| Infliximab SC 120 mg Every 4 weeks |
| Infliximab SC 180 mg Every 4 weeks |

### Estimated values for infliximab exposure parameters on infliximab SC dose and usage for each weight of RA patients

Exposure pharmacokinetic parameters (AUC_{τ}, C_{trough} and Cₘₐₓ) on infliximab SC dose and usage were simulated in such a way that a weight range of 50 - 130 kg was divided into each unit of 10 kg, wherein the estimated values for C_{trough} and AUC_{τ} of infliximab SC exposure for each weight showed a correlation with a drug dosage (Table 12).

**[Table 12]**

| **Compariso n (a)** | **N** | **Paramete** r | **C_{trough}** | | | **AUC_{τ}** | | | **Cₘₐₓ** | | |
|---|---|---|---|---|---|---|---|---|---|---|---|
| | | | **Geometr ic LS mean** | **Ratio of Geometr ic LS means** | **90% CI of Ratio (%)** | **Geometr ic LS mean** | **Ratio of Geometr ic LS means** | **90% CI of Ratio (%)** | **Geometr ic LS mean** | **Ratio of Geometr ic LS means** | **90% CI of Ratio (%)** |
| **Test: 120 mg SC at 2 weekly intervals vs Reference ^{(b)}** | | | | | | | | | | | |
| All weight | 112 5 | SC | 13.4 | 10.13 | 9.53;10.7 8 | 22492 | 1.37 | 1.33;1.40 | 18.8 | 0.25 | 0.25;0.26 |
| | | IV | 1.3 | | | 16467.2 | | | 74.8 | | |
| 50-60 kg | 100 | SC | 16.1 | 11.67 | 9.23;14.7 6 | 26855.2 | 1.69 | 1.54;1.85 | 22.4 | 0.33 | 0.31;0.35 |
| | | IV | 1.4 | | | 15893.1 | | | 68 | | |
| 60-70 kg | 100 | SC | 15.4 | 9.14 | 7.31;11.4 3 | 25450.4 | 1.44 | 1.32;1.58 | 21.1 | 0.29 | 0.27;0.31 |
| | | IV | 1.7 | | | 17660.9 | | | 73.3 | | |
| 70-80 kg | 100 | SC | 13.3 | 8.65 | 7.01;10.6 7 | 22143 | 1.25 | 1.15;1.35 | 18.4 | 0.24 | 0.22;0.25 |
| | | IV | 1.5 | | | 17751.1 | | | 77.6 | | |
| 80-90 kg | 100 | SC | 11.9 | 8.16 | 6.61;10.0 7 | 20002.3 | 1.11 | 1.02;1.20 | 16.7 | 0.21 | 0.19;0.22 |
| | | IV | 1.5 | | | 18037 | | | 81 | | |
| 90-100 kg | 100 | SC | 11 | 7.25 | 5.91;8.90 | 18518.3 | 0.99 | 0.91;1.07 | 15.5 | 0.18 | 0.17;0.20 |
| | | IV | 1.5 | | | 18754.6 | | | 84 | | |
| 100-110 kg | 100 | SC | 10.3 | 6.85 | 5.53;8.49 | 17386.5 | 0.9 | 0.82;0.98 | 14.5 | 0.16 | 0.15;0.18 |
| | | IV | 1.5 | | | 19379.7 | | | 88.3 | | |
| 110-120 kg | 100 | SC | 10.1 | 5.74 | 4.57;7.22 | 16873 | 0.82 | 0.75;0.89 | 14 | 0.16 | 0.15;0.17 |
| | | IV | 1.8 | | | 20696.5 | | | 89.8 | | |
| 120-130 kg | 100 | SC | 10 | 4.78 | 3.99;5.72 | 16620.2 | 0.75 | 0.70;0.81 | 13.8 | 0.15 | 0.14;0.16 |
| | | IV | 2.1 | | | 22191.7 | | | 94.1 | | |

| **Test: 120 mg SC at 4 weekly intervals vs Reference^{(b)}** | | | | | | | | | | | |
|---|---|---|---|---|---|---|---|---|---|---|---|
| All weight | 112 5 | SC | 4.3 | 3.24 | 3.04-3.46 | 11280.7 | 0.69 | 0.67-0.70 | 11.6 | 0.15 | 0.15;0.16 |
| | | IV | 1.3 | | | 16467.2 | | | 74.8 | | |
| 50-60 kg | 100 | SC | 5.1 | 3.8 | 3.09;4.68 | 13169.4 | 0.86 | 0.79;0.92 | 13.4 | 0.2 | 0.19;0.21 |
| | | IV | 1.3 | | | 15378.9 | | | 66.3 | | |
| 60-70 kg | 100 | SC | 4.3 | 3.57 | 2.81;4.54 | 11414.3 | 0.73 | 0.67;0.79 | 11.7 | 0.17 | 0.16;0.18 |
| | | IV | 1.2 | | | 15670.8 | | | 70.5 | | |
| 70-80 kg | 100 | SC | 4.2 | 2.93 | 2.31;3.72 | 10878.8 | 0.63 | 0.58;0.69 | 11.1 | 0.15 | 0.14;0.16 |
| | | IV | 1.4 | | | 17260.6 | | | 75.8 | | |
| 80-90 kg | 100 | SC | 4 | 2.49 | 2.02;3.07 | 10230.8 | 0.55 | 0.51;0.60 | 10.4 | 0.13 | 0.12;0.14 |
| | | IV | 1.6 | | | 18448.9 | | | 80.6 | | |
| 90-100 kg | 100 | SC | 3.2 | 2.56 | 2.05;3.19 | 8676.4 | 0.5 | 0.46;0.54 | 9 | 0.11 | 0.11;0.12 |
| | | IV | 1.2 | | | 17419.8 | | | 80.7 | | |
| 100-110 kg | 100 | SC | 3.5 | 2.01 | 1.61;2.52 | 9106.1 | 0.45 | 0.41;0.49 | 9.2 | 0.1 | 0.10;0.11 |
| | | IV | 1.8 | | | 20263.6 | | | 88.3 | | |
| 110-120 kg | 100 | SC | 3.5 | 1.74 | 1.38;2.19 | 8906 | 0.41 | 0.38;0.45 | 8.9 | 0.1 | 0.09;0.11 |
| | | IV | 2 | | | 21633 | | | 89.4 | | |
| 120-130 kg | 100 | SC | 3.2 | 1.67 | 1.34;2.07 | 8200 | 0.38 | 0.35;0.41 | 8.3 | 0.09 | 0.08;0.09 |
| | | IV | 1.9 | | | 21756.9 | | | 94.1 | | |

| **Test: 180 mg SC at 4 weekly intervals vs Reference ^{(b)}** | | | | | | | | | | | |
|---|---|---|---|---|---|---|---|---|---|---|---|
| All weight | 112 5 | SC | 6.4 | 4.86 | 4.55-5.18 | 16904.6 | 1.03 | 1.00-1.05 | 17.3 | 0.23 | 0.23;0.24 |
| | | IV | 1.3 | | | 16467.2 | | | 74.8 | | |
| 50-60 kg | 100 | SC | 7.8 | 5.77 | 4.48;7.44 | 20079.1 | 1.28 | 1.17;1.41 | 20.4 | 0.31 | 0.29;0.32 |
| | | IV | 1.3 | | | 15688.4 | | | 66.7 | | |
| 60-70 kg | 100 | SC | 6.8 | 4.94 | 4.04;6.05 | 17638 | 1.08 | 1.01;1.16 | 18 | 0.31 | 0.29;0.32 |
| | | IV | 1.4 | | | 16310.1 | | | 72.1 | | |
| 70-80 kg | 100 | SC | 5.9 | 4.66 | 3.63;5.99 | 15874.1 | 0.94 | 0.86;1.03 | 16.3 | 0.22 | 0.20;0.23 |
| | | IV | 1.3 | | | 16812 | | | 76 | | |
| 80-90 kg | 100 | SC | 5.6 | 3.97 | 3.24;4.87 | 14770.1 | 0.83 | 0.77;0.90 | 15.2 | 0.19 | 0.18;0.20 |
| | | IV | 1.4 | | | 17720.6 | | | 80.5 | | |
| 90-100 kg | 100 | SC | 5.9 | 3.11 | 2.52;3.84 | 14659.7 | 0.75 | 0.69;0.81 | 14.7 | 0.18 | 0.17;0.19 |
| | | IV | 1.9 | | | 19596.1 | | | 80.4 | | |
| 100-110 kg | 100 | SC | 5.2 | 3.04 | 2.37;3.91 | 13442.9 | 0.67 | 0.61;0.74 | 13.6 | 0.16 | 0.15;0.17 |
| | | IV | 1.7 | | | 19982.3 | | | 85.1 | | |
| 110-120 kg | 100 | SC | 4.4 | 3.04 | 2.42;3.83 | 11861.8 | 0.61 | 0.57;0.66 | 12.2 | 0.14 | 0.13;0.15 |
| | | IV | 1.5 | | | 19353.4 | | | 87.9 | | |
| 120-130 kg | 100 | SC | 4.7 | 2.58 | 2.05;3.24 | 12121.8 | 0.57 | 0.52;0.62 | 12.3 | 0.13 | 0.12;0.14 |
| | | IV | 1.8 | | | 21419.9 | | | 93.4 | | |
| Note: (a) All patients received 3 mg/kg infliximab via IV infusion over 2 hours at Weeks 0 and 2, prior to maintenance regimen commencing at Week 6 (in both test and reference periods of the cross-over). | | | | | | | | | | | |
| (b) Reference treatment comprised of 3 mg/kg every 8 weeks infliximab via IV infusion over 2 hours (maintenance regimen). | | | | | | | | | | | |
| (c) geometric LS means ratio=test/reference | | | | | | | | | | | |
| Abbreviations: CI:Confidence Interval, LS:Least Square, SD:Standard Deviation | | | | | | | | | | | |

### Example 3. Evaluation of safety and therapeutic efficacy on subcutaneously administering an infliximab to CD or UC patients

The present clinical trial of an infliximab was an open-label, randomized, multi-center, parallel-group and phase I trial designed to evaluate pharmacokinetics, efficacy and safety between the infliximab SC and the infliximab IV in patients with active CD or active UC until Week 54, wherein the present clinical trial was composed of two parts.

A part 1 was designed to find an optimal dose of the infliximab SC in CD patients, wherein the optimal dose of the infliximab SC corresponding to 5 mg/kg of the infliximab IV over the first 30 weeks was identified by means of an area under the concentration-time curve (AUC_{τ}) at steady state between Weeks 22 and 30. In case of the part 1, a clinical trial period amounted to a maximum of 65 weeks, inclusive of duration from a screening (the maximum three weeks) to End-of-Study visit.

A part 2 was designed to find that the infliximab SC was not inferior to the infliximab IV in the CD or UC patients in terms of pharmacokinetics, which would be proved by means of a concentration before the administration (C_{trough}) at Week 22. In case of the part 2, the optimal administered dose and dosing interval of the infliximab SC corresponding to 5 mg/kg of the infliximab IV were determined as follows based on the pharmacokinetics, efficacy, pharmacodynamics and safety data over the first 30 weeks of the part 1 by recommendations of the Data Safety Monitoring Board (DSMB):
- Patients weighing less than 80 kg: Administered 120 mg of the infliximab SC at an interval of two weeks; and
- Patients weighing 80 kg or more: Administered 240 mg of the infliximab SC at an interval of two weeks.

### Part 1

Patients were eligible to enroll into the present clinical trial, provided that they met all of the following criteria:
* Patient who had Crohn's disease with a score on the CDAI of 220 to 450 points;
* Patient who had Crohn's disease of at least 3 months' disease duration prior to the first administration of the study drug (Day 0); and
* Patient who had been treated for active Crohn's disease but had not responded despite a full and adequate course of therapy with a corticosteroid and/or an immunosuppressant; or who was intolerant to or had medical contraindications for such therapies.

Patients were not eligible to enroll into the clinical trial, provided that they met any one of the following criteria:
* Patient who had previously received a biological agent for the treatment of Crohn's disease or Ulcerative colitis and/or a TNFα (tumor necrosis factor-alpha) inhibitor for the treatment of other disease;
* Patient who had allergies to any of the excipients of infliximab or any other murine and/or human proteins, or patient with a hypersensitivity to immunoglobulin product;
* Patient who had active entero-vesical, entero-retroperitoneal, entero-cutaneous, and entero-vaginal fistulae within 6 months prior to the first administration of the study drug (Day 0). Entero-enteral fistulae without clinical significant symptoms upon investigator's opinion and anal fistulae without draining problems were allowed; and
* Patient who had taken more than 3 small-bowel resection procedures prior to the first administration of the study drug (Day 0).

The present clinical trial was composed of three clinical trial periods: Screening; Treatment Period; and End-of-Study. The screening was carried out between Days -21 and -1 before the initial administration of the study drug, wherein the eligibility of patients for study was evaluated. All the examinations including hepatitis B, hepatitis C and human immunodeficiency virus (HIV-1 and HIV-2) infections, a urine and serum pregnancy test for women of childbearing potential, as well as colonoscopy, CRP, 12-lead ECG, clinical laboratory tests, etc., were carried out. Also, an interferon-gamma release assay (IGRA) and a chest X-ray examination were performed so as to exclude tuberculosis (TB) patients.

Patients who met all the inclusion criteria at Week 0/Day 0 and did not correspond to any of the exclusion criteria were enrolled into the clinical trial, wherein all the enrolled patients were administered a single dose of the infliximab IV twice at Weeks 0 and 2. The patients were eligible to take following premedication at the investigator's discretion at a time point of 30 - 60 minutes before a start of the administration of the study drug so that their hypersensitivity reactions to the study drug might be prevented: e.g., antihistamine (equivalent dose of 2 - 4 mg of chlorpheniramine), hydrocortisone, paracetamol and/or nonsedating antihistamine (equivalent dose of 10 mg of cetirizine), but not limited thereto.

Those who were administered a full dose of the study drug twice and deemed to have no concerns about safety at the investigator's discretion, were randomly assigned to an infliximab SC cohort or an infliximab IV cohort before an administration at Week 6/Day 42. Such random assignment with regard to administration of the study drug was stratified by means of a region (Europe or non-Europe), Current use of treatment with azathioprine (AZA) or 6-mercaptopurine (6-MP) or methotrexate (MTX) (used or not used), a presence of clinical responses by means of CDAI-70 at Week 6, and a weight (equal to or less than 70 kg, or more than 70 kg) at Week 6. A total of 45 patients with active CD were enrolled, out of which 44 ones were randomly assigned to four clinical trial Cohorts at a ratio of 1:1:1:1, wherein the administration of the study drug was performed until Week 54 (Table 13).

**[Table 13]**

| **Cohort Number** | **Administered Dose** | **Drugs for Clinical Trials** | **Administration method** | **Number of Patients** |
|---|---|---|---|---|
| Cohort 1 | 5 m/kg | Infliximab IV 100 m/vial | 2 hours IV infusion | 13 |
| Cohort 2 | 120 mg | Infliximab SC 120 m/PFS | Single SC injection | 11 |
| Cohort 3 | 180 mg | Infliximab SC 90 m/PFS | Double SC injection | 12 |
| Cohort 4 | 240 mg | Infliximab SC 120 m/PFS | Double SC injection | 8 |

PFS, Pre-filled syringe

Those who were assigned to a cohort 1 were administered additional 7 doses of the infliximab IV at Week 6 and subsequently every eight weeks (Weeks 14, 22, 30, 38, 46 and 54). Those who were assigned to Cohorts 2, 3 and 4 were initially administered the infliximab SC at Week 6 and then additionally administered the infliximab SC every two weeks until Week 54. The initially assigned dose was adjusted to the optimal dose in all patients from Cohorts 2, 3 and 4 when the optimal dose was confirmed after dose finding. After that, an additional SC injection using the optimal dose was administered until Week 54. The infliximab SC was injected into patients by a healthcare professional at each visit (Weeks 6, 8, 10, 14, 22, 24, 26, 28, 30, 38, 46 and 54) at a study site. However, in all the other weeks (Weeks 12, 16, 18, 20, 32, 34, 36, 40, 42, 44, 48, 50 and 52), patients were allowed to perform a self-injection of the infliximab SC, if the investigator determined it as suitable after training them for appropriate injection techniques.

Patients visited the study site at a predefined time interval for clinical evaluation and blood sampling. At each visit, they were asked questions about adverse events and concomitant medication, while being monitored for clinical signs and symptoms of TB. The evaluation of primary pharmacokinetic endpoints was performed during a maintenance phase between Weeks 22 and 30, then the evaluation of secondary pharmacokinetic endpoints was performed during a treatment period until Week 54, and then blood sampling for analysis as well as the evaluation of efficacy, PD and safety were respectively performed at a point of time specified in an evaluation schedule.

The End-of-Study visit was performed in eight weeks after the end of the maintenance phase. However, it was performed in eight weeks after the last time point of administration when patients discontinued the clinical trial halfway. In case of dropout patients, all the clinical trial procedures were performed on a day of dropout or on the next day after such dropout, wherein every effort was made to complete all the End-of-Study evaluations at a time point of eight weeks after the last administration to patients.

### Part 2

A part 2 would commence based on a review by the Data Safety Monitoring Board with regard to PK modeling report data including PK, efficacy, PD and safety data, which were identified over the first 30 weeks in the part 1.

Patients would be eligible to enroll into the part 2 of the present clinical trial, provided that they met all the following criteria:
In case of active CD patients -
* Patient who had Crohn's disease with a score on the CDAI of 220 to 450 points;
* Patient who had Crohn's disease of at least 3 months' disease duration prior to the first administration of the study drug (Day 0); and
* Patient who had been treated for active Crohn's disease but had not responded despite a full and adequate course of therapy with a corticosteroid and/or an immunosuppressant; or who was intolerant to or had medical contraindications for such therapies.
* They satisfied at least one of the following items:
   - A serum C-reactive protein (CRP) concentration was more than 0.5 mg/dL;
   - A fecal calprotectin was more than 100 µg/g; and
   - Ileal-colonic CD patients had a colonoscopy (SES-CD) score of 6 points or higher in, or ileal or colonic CD patients had such score of 4 points or higher and had an ulcer score for at least one segment.

In case of active UC patients -
* Patient who had active Ulcerative colitis as defined by a total Mayo score between 6 and 12 points with endoscopic evidence of active colitis as indicated by endoscopic subscore of ≥2 at Screening;
* Patient who had Ulcerative colitis of at least 3 months' disease duration prior to the first administration of the study drug (Day 0); and
* Patient who had been treated for active Ulcerative colitis but not responded despite conventional therapy including corticosteroids alone or in combination with 6-MP or AZA and medications containing 5-ASA, or who is intolerant to or has medical contraindications for such therapies.

Patients would not be eligible to enroll into the part 2 of the clinical trial, provided that they met any one of the following criteria:
* Patient who has previously received a biological agent for the treatment of CD or UC and/or a TNFα inhibitor for the treatment of other disease;
* Patient who has allergies to any of the excipients of infliximab or any other murine and/or human proteins or patient with a hypersensitivity to immunoglobulin product;
* Patient who had active entero-vesical, entero-retroperitoneal, entero-cutaneous, and entero-vaginal fistulae within 6 months prior to the first administration of the study drug (Day 0). Entero-enteral fistulae without clinical significant symptoms upon investigator' s opinion and anal fistulae without draining problems were allowed;
* Patient who had taken more than 3 small-bowel resection procedures prior to the first administration of the study drug (Day 0); and
* Patient who had been taking rectally administered medications containing corticosteroids or 5-ASA for the treatment of ulcerative colitis within 2 weeks prior to Screening.

The part 2 would be composed of three clinical trial periods: Screening; Treatment Period; and End-of-Study. The screening would be carried out between Days -42 and 0 before an initial administration of the study drug, wherein the eligibility of patients for study would be evaluated. All the examinations including hepatitis B, hepatitis C and human immunodeficiency virus (HIV-1 and HIV-2) infections, a urine and serum pregnancy test for women of childbearing potential, as well as colonoscopy (CD patients), flexible proctosigmoidoscopy (UC patients), CRP, 12-lead ECG, clinical laboratory tests, etc., would be carried out. Also, an interferon-gamma release assay (IGRA) and a chest X-ray examination would be performed so as to exclude tuberculosis (TB) patients.

Patients who met all the inclusion criteria at Week 0/Day 0 and did not correspond to any of the exclusion criteria would be enrolled into the clinical trial, wherein all the enrolled patients would be administered a single dose of the infliximab IV twice at Weeks 0 and 2. The patients would be eligible to take following premedication at an investigator's discretion at a time point of 30 - 60 minutes before a start of the administration of the study drug so that their hypersensitivity reactions to the study drug might be prevented: e.g., antihistamine (equivalent dose of 2 - 4 mg of chlorpheniramine), hydrocortisone, paracetamol and/or nonsedating antihistamine (equivalent dose of 10 mg of cetirizine), but not limited thereto.

Those who were administered a full dose of the study drug twice and deemed to have no concerns about safety at the investigator's discretion, were randomly assigned to an infliximab SC arm or an infliximab IV arm before administration at Week 6/Day 42. Such random assignment with regard to administration of the study drug would be stratified by a current use of azathioprine or 6-mercaptopurine or MTX treatment, a disease (CD or UC), a presence of CD clinical responses by means of CDAI-70 or UC clinical responses by means of partial Mayo score at Week 6 and a weight (less than 80 kg, or 80 kg or more) at Week 6. A minimum of 130 patients with active CD or active UC would be randomly assigned to two clinical trial arms at a ratio of 1:1, wherein the administration of the study drug would be performed until Week 54 (Table 14).

**[Table 14]**

| **Arm Number** | **Administered Dose** | **Drugs for Clinical Trials** | **Administration method** | **Number of Patients** |
|---|---|---|---|---|
| Arm 1 | 5 mg/kg | infliximab IV 100 mg/vial | 2 hours IV infusion | At least 65 |
| Arm 2 | 120 mg(<80 kg) | infliximab SC 120 mg/PFS | Single SC injection | At least 65 |
| | 240 mg(≥80 kg) | infliximab SC 120 mg/PFS | Double SC injection | |

PFS, Pre-filled syringe

Those who were assigned to an arm 1 would be additionally administered the infliximab IV at Week 6 and subsequently every eight weeks (Weeks 14 and 22) until Week 22. After that, the infliximab IV would be switched to the infliximab SC administration from Week 30, wherein an SC dose would be determined based on a weight at Week 30. This dose would be administered every two weeks until Week 54. As for those who were assigned to an arm 2, an SC dose of the infliximab SC would be determined based on a weight at Week 6, wherein this dose would be administered every two weeks from Weeks 6 to 54. An increase in the dose would be permitted according to the investigator's judgment after Week 30. The infliximab SC was injected into patients by a healthcare professional at each visit (Weeks 6, 14, 22, 24, 26, 28, 30, 38, 46 and 54) at a study site. However, in all the other weeks, patients would be allowed to perform a self-injection of the infliximab SC, if the investigator determined it as suitable after training them for appropriate injection techniques.

The evaluation of primary pharmacokinetic endpoints would be performed at Week 22 and then the evaluation of secondary pharmacokinetic endpoints would be performed during a maintenance phase between Weeks 22 and 30 and during a treatment period until Week 54. Blood sampling for analysis as well as the evaluation of efficacy, PD and safety would be respectively performed at a point of time specified in an evaluation schedule.

The End-of-Study visit would be performed in two weeks after the end of the maintenance phase. However, it would be performed in two weeks after the last time point of administration when patients discontinued the clinical trial halfway after the SC administration. However, it would be performed in eight weeks after the last time point of administration when patients discontinued the clinical trial halfway after the IV administration. In case of dropout patients, all the clinical trial procedures would be performed on a day of dropout or on the next day after such dropout, wherein every effort would be made to complete all the End-of-Study evaluations at a predetermined point of time after the last administration to patients.

In case of the part 2, the clinical evaluation, blood sampling and study visits for each type would be performed in the same way as shown in the part 1 as well as at a point of time specified in an evaluation schedule.

### Results

### 3-1. Safety Evaluation

### Summary of Adverse Events

The safety evaluation was performed with regard to secondary endpoints of the part 1, i.e., immunogenicity, hypersensitivity monitoring (including delayed hypersensitivity monitoring), measurement of vital signs (including blood pressure, heart and respiration rates, and body temperature), weight, interferon-gamma release assay (IGRA), chest X-ray, hepatitis B, hepatitis C and human immunodeficiency virus (HIV-1, HIV-2) infectious states, opinions about physical examination, 12-lead ECG, adverse events (hereinafter AEs) (including serious adverse events (hereinafter SAEs)), adverse events of special interest (infusion-related reaction/hypersensitivity reaction/anaphylactic reaction[Administration-related reaction], delayed hypersensitivity reaction, injection site reaction, infection and malignancies), signs and symptoms of TB, clinical laboratory analysis, pregnancy test, prior and concomitant medication, local site pains using a 100 mm visual analogue scale (VAS), etc.

The cumulative safety data included AEs until Week 30, wherein an overall summary of AEs reported after treatment during a maintenance phase (Weeks 6 to 30) was presented in Table 15. In general, 70 TEAEs were reported in 28 patients (63.6%) - eight patients (61.5%) from the IV cohort (Cohort 1) and 20 patients (64.5%) from the SC total Cohorts (Cohorts 2 - 4), indicating that the proportion was similar between the two groups. Also, the intensity of most TEAEs was shown as a grade 1 or 2. Among all the TEAEs, the AEs reported in nine patients (20.5%) were regarded to be related to the study drug.

The treatment-emergent serious AEs (hereinafter TESAEs) were reported in eight patients (18.2%) - two patients (15.4%) from the IV cohort (Cohort 1) and six patients (19.4%) from the SC total Cohorts (Cohorts 2 - 4). All the TESAEs were regarded not to be associated with the study drug.

Out of the TEAEs classified as ARRs, the infusion-related reaction, hypersensitivity or anaphylactic reactions were reported for one patient (the very day of medication at Week 30) in the IV cohort (Cohort 1) and one patient (two days after medication at Week 6) in the SC cohort (Cohort 4). As an infliximab administration-related reaction was associated with an anti-drug antibody (ADA) (Remsima SmPC 2017), the presence of the ADA was assessed for patients having shown the administration-related reaction. Patients who had positive results for the ADA and a neutralizing antibody (NAb) amounted to just one (IV cohort (Cohort 1)), wherein the patient took a medication of an IV drug in a maintenance phase after a dose loading period, i.e., at Weeks 6 and 14, then was identified as ADA positive from Week 22, and then the administration-related reaction was reported at Week 30.

The TEAEs classified as an injection site reaction were reported in four patients (12.9%) of the SC cohort (Cohorts 2 - 4), wherein the intensity thereof all was shown as a grade 1 or 2. The presence of the ADA was evaluated even for patients having shown the injection site reaction, wherein two of the four patients have positive results for the ADA and the NAb during the clinical trial period.

The TEAEs classified as an infection were reported in two patients (15.4%) of the IV cohort (Cohort 1) and seven patients (22.6%) of the SC cohort (Cohorts 2 - 4).

TEAEs leading to discontinuation of study drug were reported for one patient (7.7%) in the IV cohort (Cohort 1) and four patients (12.9%) in the SC cohort (Cohorts 2 - 4), out of which only one patient of the cohort 3 discontinued the administration of the study drug due to the TEAEs regarded to be associated with the study drug, which was the latent TB of the intensity grade 1.

Death reported during the clinical trial period was two cases (each one from Cohorts 1 and 3). One patient of the IV cohort (Cohort 1) died from sudden cardiac death at home in one month after the completion of medication at Week 6, which was not reportedly associated with the study drug. Such patient had comorbidity of hypertension and chronic heart failure. In the SC cohort (Cohort 3), one patient died from sudden death at home at the very night after medication at Week 30, which was not reportedly associated with the study drug. Such patient had comorbidity of chronic aortic calcification, diabetes, obesity and metabolic syndrome.

**[Table 15]**

| | **Cohort 1 IV 5 mg/kg (N=13)** | **Cohort 2 SC 120 mg (N=11)** | **Cohort 3 SC 180 mg (N=12)** | **Cohort 4 SC 240 mg (N=8)** | **Total SC (N=31)** |
|---|---|---|---|---|---|
| Number of Patients with at Least one TEAE (%) | 8 (61.5) | 8 (72.7) | 7 (58.3) | 5 (62.5) | 20 (64.5) |
| Related | 4 (30.8) | 1 (9.1) | 1 (8.3) | 3 (37.5) | 5 (16.1) |
| Unrelated | 6 (46.2) | 7 (63.6) | 7 (58.3) | 4 (50.0) | 18 (58.1) |
| Number of Patients with at Least one TESAE (%) | 2 (15.4) | 2 (18.2) | 1 (8.3) | 3 (37.5) | 6 (19.4) |
| Related | 0 | 0 | 0 | 0 | 0 |
| Unrelated | 2 (15.4) | 2 (18.2) | 1 (8.3) | 3 (37.5) | 6 (19.4) |
| Number of Patients with at Least One TEAE classified as administration-related reactions (%) | 1 (7.7) | 0 | 0 | 1 (12.5) | 1 (3.2) |
| Number of Patients with at Least One TEAE classified as Injection Site Reaction (%) | 0 | 1 (9.1) | 2 (16.7) | 1 (12.5) | 4 (12.9) |
| Number of Patients with at Least One TEAE classified as infection (%) | 2 (15.4) | 4 (36.4) | 1 (8.3) | 2 (25.0) | 7 (22.6) |
| Related | 1 (7.7) | 0 | 1 (8.3) | 1 (12.5) | 2 (6.5) |
| Number of Patients with at Least One TEAE Leading to Study Drug Discontinuation (%) | 1 (7.7) | 1 (9.1) | 2 (16.7) | 1 (12.5) | 4 (12.9) |

* At each level of summarization, patients were counted once if they reported one or more events. Only the most severe event is counted. The event is considered to be related if the relationship is defined as 'Possible', 'Probable', 'Definite'.

### Immunogenicity

In general, the proportion of patients who had positive results for the ADA was low in the SC Cohorts, while most patients tested negative for the ADA at Week 30. The number of patients who had positive results for the ADA at Week 30 amounted to 8 (61.5%), 0 (0.0%), 3 (25.0%) and 1 (12.5%) in the Cohorts 1 to 4, respectively (Table 16).

**[Table 16]**

| **Visit** | **Cohort 1 IV 5 mg/kg (N=13)** | **Cohort 2 SC 120 mg (N=11)** | **Cohort 3 SC 180 mg (N=12)** | **Cohort 4 SC 240 mg (N=8)** | **Total SC (N=31)** |
|---|---|---|---|---|---|
| Week 0 | | | | | |
| ADA positive | 0 | 0 | 0 | 1 (12.5) | 1 (3.2) |
| NAb positive | 0 | 0 | 0 | 0 | 0 |

| Week 6 | | | | | |
|---|---|---|---|---|---|
| ADA positive | 1 (7.7) | 0 | 0 | 3 (37.5) | 3 (9.7) |
| NAb positive | 1 (7.7) | 0 | 0 | 2 (25.0) | 2 (6.5) |

| Week 14 | | | | | |
|---|---|---|---|---|---|
| ADA positive | 4 (30.8) | 0 | 1 (8.3) | 2 (25.0) | 3 (9.7) |
| NAb positive | 2 (15.4) | 0 | 1 (8.3) | 1 (12.5) | 2 (6.5) |

| Week 22 | | | | | |
|---|---|---|---|---|---|
| ADA positive | 7 (53.8) | 0 | 1 (8.3) | 2 (25.0) | 3 (9.7) |
| NAb positive | 5 (38.5) | 0 | 1 (8.3) | 2 (25.0) | 3 (9.7) |

| Week 30 | | | | | |
|---|---|---|---|---|---|
| ADA positive | 8 (61.5) | 0 | 3 (25.0) | 1 (12.5) | 4 (12.9) |
| NAb positive | 4 (30.8) | 0 | 1 (8.3) | 1 (12.5) | 2 (6.5) |
| at least one ADA positive* after 6 week | 7 (58.3) | 0 | 3 (25.0) | 0 | 3 (9.7) |
| at least one NAb positive* after 6 week | 5 (41.7) | 0 | 1 (8.3) | 0 | 1(3.2) |

| | | | | | |
|---|---|---|---|---|---|
| * ADA, Anti-drug antibody; NAb, Neutralizing antibody ** The percentage of visits was calculated using the number of randomly assigned patients as the denominator. * Denominator: Number of ADA negative patients at 0 and 6 weeks; numerator: Number of ADA or NAB positive patients at 14 and 30 weeks | | | | | |

### Evaluation of local site pains using a visual analogue scale (VAS)

The range of the VAS amounted to 0 to 100 mm, with a higher score indicating a more severe pain. In the Cohorts 3 and 4, in which two injections had to be performed in each visit, a slightly higher level of local site pains was observed than in other Cohorts. In general, a low level of local site pains (same or less than 21.05 mm on average) was observed in all the Cohorts (Table 17).

**[Table 17]**

| **Visit Statistic** | **Cohort 1 IV 5 mg/kg (N=13)** | **Cohort 2 SC 120 mg (N=11)** | **Cohort 3 SC 180 mg (N=12)** | **Cohort 4 SC 240 mg (N=8)** |
|---|---|---|---|---|
| Week 6 | | | | |
| n | 13 | 11 | 12 | 8 |
| Mean (SD) | 16.32 (18.929) | 8.43 (7.807) | 21.87 (26.124) | 19.31 (18.676) |
| Median | 7 | 7 | 11.85 | 11.5 |
| Minimum, Maximum | 0,67 | 0,25 | 0,79.2 | 6,63 |

| Week 14 | | | | |
|---|---|---|---|---|
| n | 12 | 10 | 12 | 6 |
| Mean (SD) | 6.38 (8.742) | 5.60 (4.203) | 11.93 (13.145) | 18.75 (22.018) |
| Median | 2.25 | 6 | 6.65 | 8.5 |
| Minimum, Maximum | 0,28 | 0,13 | 0,39 | 3,59 |

| Week 22 | | | | |
|---|---|---|---|---|
| n | 11 | 10 | 11 | 7 |
| Mean (SD) | 4.23 (5.574) | 10.10 (12.838) | 14.15 (13.828) | 12.07 (10.675) |
| Median | 2 | 6 | 9 | 10 |
| Minimum, Maximum | 0,15 | 0,45.5 | 0,40 | 3,34.5 |

| Week 30 | | | | |
|---|---|---|---|---|
| n | 10 | 9 | 11 | 5 |
| Mean (SD) | 4.69 (6.872) | 9.08 (8.851) | 21.05 (21.887) | 12.60 (10.158) |
| Median | 2.7 | 9 | 12 | 8 |
| Minimum, Maximum | 0,23.5 | 0,29.1 | 1,71 | 3.5,29 |

### 3-2. Therapeutic efficacy evaluation

### Disease activity measured by CDAI

As a result of analyzing the CDAI (Crohn's disease activity index) scores of Weeks 2, 6, 14, 22 and 30 in comparison with baseline as a secondary efficacy endpoint, it was identified that actual values tended to decrease in each cohort as time went by. The proportion of patients responding to CDAI-70 criteria at Week 6, one of stratified randomization variables, was slightly higher in the cohort 2 than in other Cohorts, wherein accordingly more attentions were required to be paid to interpretation of efficacy results.

The actual values of the disease activity measured by the CDAI as well as the changed values from baseline were summarized in Table 18.

**[Table 18]**

| | **IV 5mg/kg (N=12)** | | **SC 120mg (N=11)** | | **SC 180mg (N=12)** | | **SC 240mg (N=7)** | |
|---|---|---|---|---|---|---|---|---|
| **Visit Statistic** | **Actual Result** | **Change from Baseline** | **Actual Result** | **Change from Baseline** | **Visit Statistic** | **Actual Result** | **Change from Baseline** | **Actual Result** |
| Baseline | | | | | | | | |
| n | 12 | | 11 | | 12 | | 7 | |
| Mean | 310.13 | | 298.16 | | 302.38 | | 324.36 | |
| SD | 73.353 | | 56.490 | | 73.633 | | 47.866 | |
| Minimum | 221.8 | | 228.8 | | 229.1 | | 249.9 | |
| Median | 279.35 | | 290.10 | | 266.75 | | 317.40 | |
| Maximum | 433.5 | | 409.2 | | 422.5 | | 394.9 | |

| Week 2 | | | | | | | | |
|---|---|---|---|---|---|---|---|---|
| n | 12 | 12 | 11 | 11 | 12 | 12 | 7 | 7 |
| Mean | 243.01 | -67.13 | 219.88 | -78.28 | 266.96 | -35.43 | 254.59 | -69.77 |
| SD | 97.757 | 59.875 | 99.370 | 72.811 | 52.401 | 67.369 | 90.915 | 69.656 |
| Minimum | 48.8 | -173.0 | 120.9 | -169.6 | 175.0 | -163.4 | 117.5 | -199.9 |
| Median | 227.05 | -57.10 | 174.70 | -77.70 | 269.75 | -39.25 | 228.90 | 42.00 |
| Maximum | 394.1 | 72.0 | 457.8 | 48.6 | 357.8 | 86.0 | 381.0 | -7.8 |

| Week 6 | | | | | | | | |
|---|---|---|---|---|---|---|---|---|
| n | 12 | 12 | 11 | 11 | 12 | 12 | 7 | 7 |
| Mean | 212.20 | -97.93 | 172.58 | -125.58 | 216.43 | -85.96 | 220.34 | -104.01 |
| SD | 92.019 | 98.599 | 105.719 | 115.909 | 70.507 | 82.388 | 79.041 | 61.204 |
| Minimum | 56.2 | -211.1 | 47.6 | -268.0 | 28.6 | -209.4 | 103.5 | -213.9 |
| Median | 230.65 | -145.40 | 149.20 | -141.70 | 240.20 | -88.25 | 215.30 | -88.40 |
| Maximum | 371.7 | 102.2 | 365.4 | 102.3 | 280.5 | 42.0 | 340.5 | -34.6 |

| Week 14 | | | | | | | | |
|---|---|---|---|---|---|---|---|---|
| n | 12 | 12 | 10 | 10 | 12 | 12 | 7 | 7 |
| Mean | 192.33 | -117.81 | 138.86 | -156.55 | 188.41 | -113.98 | 216.24 | -108.11 |
| SD | 96.466 | 103.053 | 97.259 | 128.873 | 82.635 | 109.633 | 108.773 | 105.399 |
| Minimum | 5.0 | -259.0 | 49.0 | -360.2 | 27.0 | -328.3 | 54.8 | -252.4 |
| Median | 203.55 | -101.95 | 89.40 | -151.00 | 206.10 | -101.70 | 258.40 | -85.30 |
| Maximum | 346.0 | 76.5 | 334.6 | 61.3 | 276.5 | 23.4 | 355.8 | 17.6 |

| Week 22 | | | | | | | | |
|---|---|---|---|---|---|---|---|---|
| n | 11 | 11 | 10 | 10 | 11 | 11 | 7 | 7 |
| Mean | 145.25 | -168.58 | 126.86 | -168.55 | 155.41 | -136.05 | 159.67 | -164.69 |
| SD | 103.340 | 107.225 | 78.765 | 109.101 | 66.512 | 90.717 | 124.519 | 117.773 |
| Minimum | -11.8 | -369.6 | 35.6 | -312.2 | 23.6 | -314.0 | 22.6 | -293.2 |
| Median | 154.40 | -133.00 | 107.20 | -164.15 | 164.00 | -106.40 | 158.20 | -198.40 |
| Maximum | 348.1 | -32.3 | 304.2 | 41.1 | 252.9 | -0.2 | 317.2 | 7.3 |

| Week 30 | | | | | | | | |
|---|---|---|---|---|---|---|---|---|
| n | 10 | 10 | 9 | 9 | 11 | 11 | 6 | 6 |
| Mean | 138.10 | -178.92 | 83.37 | -215.63 | 121.38 | -170.08 | 136.12 | -191.07 |
| SD | 109.844 | 105.117 | 40.112 | 62.241 | 70.646 | 79.501 | 96.749 | 87.759 |
| Minimum | -16.0 | -355.6 | 21.4 | -299.0 | 17.8 | -265.0 | 19.6 | -297.8 |
| Median | 125.40 | -156.95 | 73.40 | -199.50 | 125.80 | -178.40 | 135.65 | -211.70 |
| Maximum | 397.4 | -36.1 | 138.5 | -107.9 | 274.1 | 21.0 | 306.6 | -68.4 |

### Response evaluation according to CDAI-70 and CDAI-100 response criteria

The proportion of patients responding to CDAI-70 response criteria based on CDAI scores (those having a decrease in CDAI scores by 70 points or more from the baseline value) showed a similar level among respective treatment groups (Table 19). The proportion of patients responding to CDAI-100 response criteria (those having a decrease in CDAI scores by 100 points or more from the baseline value) also showed a similar level among respective treatment groups (Table 20).

**[Table 19]**

| **Visit Statistic** | **IV 5 mg/kg (N=12)** | **SC 120 mg (N=11)** | **SC 180 mg (N=12)** | **SC 240 mg (N=7)** |
|---|---|---|---|---|
| Week 2 | | | | |
| Number of patients responded (%) | 5 (41.7) | 7 (63.6) | 3 (25.0) | 3 (42.9) |

| Week 6 | | | | |
|---|---|---|---|---|
| Number of patients responded (%) | 7 (58.3) | 9 (81.8) | 7 (58.3) | 5 (71.4) |

| Week 14 | | | | |
|---|---|---|---|---|
| Number of patients responded (%) | 8 (66.7) | 8 (72.7) | 8 (66.7) | 4 (57.1) |

| Week 22 | | | | |
|---|---|---|---|---|
| Number of patients responded (%) | 9 (75.0) | 9 (81.8) | 9 (75.0) | 5 (71.4) |

| Week 30 | | | | |
|---|---|---|---|---|
| Number of patients responded (%) | 8 (66.7) | 9 (81.8) | 10 (83.3) | 5 (71.4) |

**[Table 20]**

| **Visit Statistic** | **IV 5 mg/kg (N=12)** | **SC 120 mg (N=11)** | **SC 180 mg (N=12)** | **SC 240 mg (N=7)** |
|---|---|---|---|---|
| Week 2 | | | | |
| Number of patients responded (%) | 3 (25.0) | 5 (45.5) | 2 (16.7) | 3 (42.9) |

| Week 6 | | | | |
|---|---|---|---|---|
| Number of patients responded (%) | 7 (58.3) | 6 (54.5) | 5 (41.7) | 3 (42.9) |

| Week 14 | | | | |
|---|---|---|---|---|
| Number of patients responded (%) | 6 (50.0) | 6 (54.5) | 6 (50.0) | 3 (42.9) |

| Week 22 | | | | |
|---|---|---|---|---|
| Number of patients responded (%) | 8 (66.7) | 7 (63.6) | 7 (58.3) | 4 (57.1) |

| Week 30 | | | | |
|---|---|---|---|---|
| Number of patients responded (%) | 7 (58.3) | 9 (81.8) | 10 (83.3) | 5 (71.4) |

### Clinical remission evaluation

The proportion of patients achieving a clinical remission (those having an absolute CDAI score of less than 150 point) was similar among the treatment groups. A comparatively high proportion of patients achieving the clinical remission was observed in Cohort 2 during a medication maintenance period (Weeks 6 to 30), but six patients (54.5%) of Cohort 2 were already in a state of having achieved the clinical remission at Week 6, while most of these patients maintained a state of the clinical remission until Week 30 (Table 21).

**[Table 21]**

| **Visit Statistic** | **IV 5 mg/kg (N=12)** | **SC 120 mg (N=11)** | **SC 180 mg (N=12)** | **SC 240 mg (N=7)** |
|---|---|---|---|---|
| Week 2 | | | | |
| Number of patients achieved (%) | 2 (16.7) | 2 (18.2) | 0 | 1 (14.3) |

| Week 6 | | | | |
|---|---|---|---|---|
| Number of patients achieved (%) | 3 (25.0) | 6 (54.5) | 2 (16.7) | 1 (14.3) |

| Week 14 | | | | |
|---|---|---|---|---|
| Number of patients achieved (%) | 3 (25.0) | 6 (54.5) | 4 (33.3) | 2 (28.6) |

| Week 22 | | | | |
|---|---|---|---|---|
| Number of patients achieved (%) | 5 (41.7) | 7 (63.6) | 4 (33.3) | 3 (42.9) |

| Week 30 | | | | |
|---|---|---|---|---|
| Number of patients achieved (%) | 7 (58.3) | 9 (81.8) | 7 (58.3) | 5 (71.4) |

### Example 4. Modeling for subcutaneously administering an infliximab to CD patients

### PK-PD Model Construction

A PK-PD model construction was performed in the same way as shown in a method of Example 2.

### Dose-regimen scenario of SC dosage form for modeling in CD patients

A pharmacokinetic aspect was predicted based on a following simulation scenario, wherein accordingly efficacy and safety were also predicted, and then evaluated in comparison with an infliximab IV infusion usage, in which a maintenance dose of 5 mg/kg was administered at an interval of eight weeks (Table 22).

**[Table 22]**

| |
|---|
| Infliximab SC 120 mg Every 2 weeks |
| Infliximab SC 180 mg Every 2 weeks |
| Infliximab SC 240 mg Every 2 weeks |

### Estimated values for infliximab exposure parameters on infliximab SC dose and usage for each weight of CD patients

Exposure pharmacokinetic parameters on infliximab SC dose and usage were simulated in such a way that a weight range of 50 - 130 kg was divided into each unit of 10 kg, wherein the estimated values for a minimum concentration immediately before the next study drug administration (C_{trough}) and an area under the concentration-time curve (AUC_{τ}) of SC exposure showed a correlation with a drug dosage (Table 23).

**[Table 23]**

| **Compariso n(a)** | **N** | **Paramete r** | **C_{trough}** | | | **AUC_{τ}** | | | **Cₘₐₓ** | | |
|---|---|---|---|---|---|---|---|---|---|---|---|
| | | | **Geometr ic LS mean** | **Ratio of Geometr ic LS means** | **90% CI of Ratio (%)** | **Geometr ic LS mean** | **Ratio of Geometr ic LS means** | **90% CI of Ratio (%)** | **Geometr ic LS mean** | **Ratio of Geometr ic LS means** | **90% CI of Ratio (%)** |
| **Test: 120 mg SC at 2 weekly intervals vs Reference ^{(b)}** | | | | | | | | | | | |
| All weight | 265 | SC | 12.8 | 7.76 | 6.57;9.16 | 21591.7 | 0.9 | 0.84;0.95 | 18 | 0.18 | 0.17;0.18 |
| | | IV | 1.7 | | | 24093.4 | | | 102.3 | | |
| 50-60 kg | 100 | SC | 15.8 | 8.14 | 6.39;10.3 6 | 26409.1 | 1.05 | 0.96;1.15 | 21.9 | 0.19 | 0.18;0.21 |
| | | IV | 1.9 | | | 25184.2 | | | 112.4 | | |
| 60-70 kg | 100 | SC | 12.8 | 8.43 | 6.60;10.7 5 | 21721.4 | 0.88 | 0.80;0.96 | 18.2 | 0.15 | 0.14;0.16 |
| | | IV | 1.5 | | | 24685.5 | | | 119.6 | | |
| 70-80 kg | 100 | SC | 12.3 | 6.68 | 5.26;8.48 | 20668.2 | 0.76 | 0.70;0.83 | 17.2 | 0.13 | 0.13;0.14 |
| | | IV | 1.8 | | | 27245.1 | | | 129 | | |
| 80-90 kg | 100 | SC | 11.3 | 6.18 | 4.75;8.03 | 19046.9 | 0.67 | 0.61;0.74 | 15.9 | 0.12 | 0.11;0.13 |
| | | IV | 1.8 | | | 28287.6 | | | 134.3 | | |
| 90-100 kg | 100 | SC | 11.2 | 4.93 | 3.82;6.37 | 18698.2 | 0.6 | 0.55;0.66 | 15.5 | 0.11 | 0.10;0.12 |
| | | IV | 2.3 | | | 30916.4 | | | 139.7 | | |
| 100-110 kg | 100 | SC | 10.3 | 4.64 | 3.59;5.99 | 17331.1 | 0.54 | 0.49;0.60 | 14.4 | 0.1 | 0.09;0.10 |
| | | IV | 2.2 | | | 31999.9 | | | 149 | | |
| 110-120 kg | 100 | SC | 9.9 | 4.17 | 3.20;5.42 | 16654.4 | 0.5 | 0.45;0.55 | 13.9 | 0.09 | 0.08;0.10 |
| | | IV | 2.4 | | | 33437.4 | | | 152.6 | | |
| 120-130 kg | 100 | SC | 9 | 4.43 | 3.33;5.90 | 15388.5 | 0.46 | 0.41;0.51 | 12.9 | 0.08 | 0.07;0.09 |
| | | IV | 2 | | | 33509.7 | | | 161.9 | | |

| **Test: 180 mg SC at 2 weekly intervals vs Reference ^{(b)}** | | | | | | | | | | | |
|---|---|---|---|---|---|---|---|---|---|---|---|
| All weight | 265 | SC | 19.2 | 11.62 | 9.85;13.7 2 | 32367.4 | 1.34 | 1.26;1.43 | 26.9 | 0.26 | 0.25;0.28 |
| | | IV | 1.7 | | | 24093.4 | | | 102.3 | | |
| 50-60 kg | 100 | SC | 23.7 | 12.19 | 9.57;15.5 1 | 39576.4 | 1.57 | 1.44;1.72 | 32.8 | 0.29 | 0.27;0.31 |
| | | IV | 1.9 | | | 25184.2 | | | 112.4 | | |
| 60-70 kg | 100 | SC | 19.2 | 12.62 | 9.90;16.1 1 | 32541.4 | 1.32 | 1.20;1.45 | 27.2 | 0.23 | 0.21;0.24 |
| | | IV | 1.5 | | | 24685.5 | | | 119.6 | | |
| 70-80 kg | 100 | SC | 18.4 | 10.01 | 7.88;12.7 2 | 30981 | 1.14 | 1.05;1.24 | 25.8 | 0.2 | 0.19;0.21 |
| | | IV | 1.8 | | | 27245.1 | | | 129 | | |
| 80-90 kg | 100 | SC | 16.9 | 9.26 | 7.13;12.0 4 | 28541.7 | 1.01 | 0.92;1.11 | 23.8 | 0.18 | 0.16;0.19 |
| | | IV | 1.8 | | | 28287.6 | | | 134.3 | | |
| 90-100 kg | 100 | SC | 16.7 | 7.39 | 5.72;9.54 | 28000.4 | 0.91 | 0.82;0.99 | 23.2 | 0.17 | 0.15;0.18 |
| | | IV | 2.3 | | | 30916.4 | | | 139.7 | | |
| 100-110 kg | 100 | SC | 15.4 | 6.95 | 5.38;8.98 | 25959 | 0.81 | 0.74;0.89 | 21.6 | 0.14 | 0.14;0.16 |
| | | IV | 2.2 | | | 31999.9 | | | 149 | | |
| 110-120 kg | 100 | SC | 14.9 | 6.25 | 4.80;8.13 | 24932.8 | 0.75 | 0.68;0.82 | 20.7 | 0.14 | 0.13;0.15 |
| | | IV | 2.4 | | | 33437.4 | | | 152.6 | | |
| 120-130 kg | 100 | SC | 13.5 | 6.64 | 4.98;8.84 | 23025 | 0.69 | 0.62;0.76 | 19.3 | 0.12 | 0.11;0.13 |
| | | IV | 2 | | | 33509.7 | | | 161.9 | | |

| **Test: 240 mg SC at 2 weekly intervals vs Reference^{(b)}** | | | | | | | | | | | |
|---|---|---|---|---|---|---|---|---|---|---|---|
| All weight | 265 | SC | 25.6 | 15.49 | 13.12;18. 28 | 43143.1 | 1.79 | 1.68;1.91 | 35.9 | 0.35 | 0.33;0.37 |
| | | IV | 1.7 | | | 24093.4 | | | 102.3 | | |
| 50-60 kg | 100 | SC | 31.6 | 16.23 | 12.75;20. 66 | 52743.5 | 2.09 | 1.91;2.29 | 43.7 | 0.39 | 0.36;0.42 |
| | | IV | 1.9 | | | 25184.2 | | | 112.4 | | |
| 60-70 kg | 100 | SC | 25.5 | 16.81 | 13.18;21. 44 | 43361.1 | 1.76 | 1.60;1.93 | 36.3 | 0.3 | 0.28;0.33 |
| | | IV | 1.5 | | | 24685.5 | | | 119.6 | | |
| 70-80 kg | 100 | SC | 24.5 | 13.34 | 10.50;16. 94 | 41293.8 | 1.52 | 1.39;1.65 | 34.4 | 0.27 | 0.25;0.28 |
| | | IV | 1.8 | | | 27245.1 | | | 129 | | |
| 80-90 kg | 100 | SC | 22.5 | 12.34 | 9.49;16.0 | 38036.3 | 1.34 | 1.22;1.48 | 31.7 | 0.24 | 0.22;0.25 |
| | | IV | 1.8 | | 4 | 28287.6 | | | 134.3 | | |
| 90-100 kg | 100 | SC | 22.3 | 9.85 | 7.62;12.7 2 | 37302 | 1.21 | 1.10;1.32 | 31 | 0.22 | 0.21;0.24 |
| | | IV | 2.3 | | | 30916.4 | | | 139.7 | | |
| 100-110 kg | 100 | SC | 20.6 | 9.26 | 7.17;11.9 6 | 34586.7 | 1.08 | 0.98;1.19 | 28.8 | 0.19 | 0.18;0.21 |
| | | IV | 2.2 | | | 31999.9 | | | 149 | | |
| 110-120 kg | 100 | SC | 19.8 | 8.33 | 6.40;10.8 3 | 33210.9 | 0.99 | 0.90;1.10 | 27.6 | 0.18 | 0.17;0.19 |
| | | IV | 2.4 | | | 33437.4 | | | 152.6 | | |
| 120-130 kg | 100 | SC | 18.0 | 8.84 | 6.64;11.7 8 | 30661.1 | 0.91 | 0.83;1.01 | 25.7 | 0.16 | 0.15;0.17 |
| | | IV | 2 | | | 33509.7 | | | 161.9 | | |
| Note: (a) All patients received 5 mg/kg infliximab via IV infusion over 2 hours at weeks 0 and 2, prior to maintenance regimen commencing at week 6 (in both test and reference periods of the cross-over). | | | | | | | | | | | |
| (b) Reference treatment comprised of 5 mg/kg every 8 weeks infliximab via IV infusion over 2 hours (maintenance regimen). | | | | | | | | | | | |
| (c) geometric LS means ratio=test/reference | | | | | | | | | | | |
| Abbreviations: CI:Confidence Interval, LS:Least Square, SD:Standard Deviation | | | | | | | | | | | |

## Claims

1. A method for treatment of TNF-α-related disease, comprising a step of administering to a patient a pharmaceutical composition comprising an anti-TNF-α antibody or its antigen binding fragment, wherein the anti-TNF-α antibody or its antigen binding fragment is administered subcutaneously to the patient at a dose of 60 to 300 mg and at intervals of 1 to 8 weeks.

2. The method of claim 1, wherein the TNF-α-related disease is selected from the group consisting of rheumatoid arthritis, ulcerative colitis, Crohn's disease, plaque psoriasis, psoriatic arthritis, and ankylosing spondylitis.

3. The method of claim 2, wherein the TNF-α-related disease is rheumatoid arthritis.

4. The method of claim 3, wherein the anti-TNF-α antibody or its antigen binding fragment is administered to the patient at a dose of 90 to 180 mg.

5. The method of claim 2, wherein the TNF-α-related disease is selected from the group consisting of ulcerative colitis, Crohn's disease, plaque psoriasis, psoriatic arthritis, and ankylosing spondylitis.

6. The method of claim 5, wherein the anti-TNF-α antibody or its antigen binding fragment is administered to the patient at a dose of 120 to 240 mg.

7. The method of claim 1, wherein the anti-TNF-α antibody or its antigen binding fragment is administered to the patient at a dose of 80 to 100 mg, 110 to 130 mg, 170 to 190 mg, or 230 to 250 mg.

8. The method of claim 7, wherein the anti-TNF-α antibody or its antigen binding fragment is administered to the patient at a dose of 90 mg, 120 mg, 180 mg or 240 mg.

9. The method of claim 1, further comprising a step of determining the dose according to the body weight of the patient, wherein the anti-TNF-α antibody or its antigen binding fragment is administered at a dose of 90 to 180 mg when the body weight of the patient is less than 80 kg, and is administered at a dose of 190 to 270 mg when the body weight of the patient is more than 80 kg.

10. The method of claim 1, wherein the anti-TNF-α antibody or its antigen binding fragment is administered to the patient at intervals of 1, 2, 3, 4, 5, 6, 7 or 8 weeks.

11. The method of claim 10, wherein the anti-TNF-α antibody or its antigen binding fragment is administered to the patient at intervals of 2 or 4 weeks.

12. The method of claim 1, wherein the anti-TNF-α antibody or its antigen binding fragment is co-administered with a disease-modifying anti rheumatic drug (DMARD).

13. The method of claim 12, wherein the disease-modifying anti rheumatic drug (DMARD) is selected from the group consisting of methotrexate, leflunomide, sulfasalazine and hydroxychloroquine.

14. The method of claim 1, wherein the patient is a patient who has been administered at least once intravenously with the anti-TNF-α antibody or its antigen-binding fragment before the subcutaneous administration.

15. The method of claim 14, wherein the patient is a patient who has been administered intravenously with the anti-TNF-α antibody or its antigen-binding fragment at a dose of 1 to 10 mg/kg for each administration.

16. The method of claim 14, wherein the first subcutaneous administration is performed 2 to 8 weeks after the last intravenous administration.

17. The method of claim 1, wherein the anti-TNF-α antibody or its antigen-binding fragment is maintained at a minimum blood concentration (C_{trough}) of 3 to 16 µg/mL after it is administered subcutaneously to the patient.

18. The method of claim 1, wherein the anti-TNF-α antibody or its antigen-binding fragment is maintained at a minimum blood concentration (C_{trough}) of 9 to 32 µg/mL after it is administered subcutaneously to the patient.

19. The method of claim 1, wherein the patient after the subcutaneous administration has one or more of the following characteristics:
a) a decrease in DAS28 (Disease Activity Score in 28 joints) of at least 2.0; or
b) a decrease in CDAI (Crohn's disease activity index) of at least 70.

20. The method of claim 1, wherein the patient before the subcutaneous administration has one or more of the following characteristics:
a) a patient who has an inadequate response to disease-modifying anti rheumatic drugs (DMARDs), including methotrexate;
b) a patient who has not previously been treated with methotrexate and other DMARDs;
c) a patient who exhibits elevated serologic indicators associated with severe axial-predominant symptoms and inflammation, which show no proper response to common therapies; or
d) a patient who does not respond to, or is contraindicated to, or has intolerance to methotrexate, cyclosporine, or systemic therapies including psoralen ultraviolet A therapy (PUVA).

21. The method of claim 1, wherein the patient before the subcutaneous administration has one or more of the following characteristics:
a) a patient who has an inadequate response to, or has intolerance to, or is contraindicated for treatment with corticosteroids, 6-mercaptopurine, azathioprine or immunosuppressants; or
b) a patient who does not respond to common therapies, including antibiotic, excretion or immunosuppressive therapies.

22. The method of claim 1, wherein the anti-TNF-α antibody or its antigen-binding fragment comprises:
a light-chain variable region comprising a CDR1 domain comprising an amino acid sequence of SEQ ID NO: 1, a CDR2 domain comprising an amino acid sequence of SEQ ID NO: 2, and a CDR3 domain comprising an amino acid sequence of SEQ ID NO: 3; and
a heavy-chain variable region comprising a CDR1 domain comprising an amino acid sequence of SEQ ID NO: 4, a CDR2 domain comprising an amino acid sequence of SEQ ID NO: 5, and a CDR3 domain comprising an amino acid sequence of SEQ ID NO: 6.

23. The method of claim 1, wherein the anti-TNF-α antibody or its antigen-binding fragment comprises:
a light-chain variable region comprising an amino acid sequence of SEQ ID NO: 7; and
a heavy-chain variable region comprising an amino acid sequence of SEQ ID NO: 8.

24. The method of claim 1, wherein the anti-TNF-α antibody or its antigen-binding fragment comprises:
a light chain comprising an amino acid sequence of SEQ ID NO: 9; and
a heavy chain comprising an amino acid sequence of SEQ ID NO: 10.

25. The method of claim 1, wherein the anti-TNF-α antibody is infliximab.

26. The method of claim 1, wherein the composition comprising the anti-TNF-α antibody or its antigen binding fragment contains: (A) 90 to 180 mg/ml of the anti-TNFα antibody or its antibody binding fragment; (B) 0.02 to 0.1 % (w/v) of polysorbate; (C) 1 to 10% (w/v) of sorbitol; and (D) 1 to 50 mM of a buffer comprising acetate.

27. The method of claim 1, wherein the composition comprising the anti-TNF-α antibody or its antigen binding fragment is filled in a pre-filled syringe or an auto-injector before administration to the patient.

28. A pharmaceutical composition for treatment of TNF-α-related disease, comprising an anti-TNF-α antibody or its antigen binding fragment, wherein the anti-TNF-α antibody or its antigen binding fragment is to be administered subcutaneously at a dose of 60 to 300 mg and at intervals of 1 to 8 weeks.

29. A kit comprising:
(a) a pharmaceutical composition comprising an anti-TNF-α antibody or its antigen binding fragment; and
(b) instructions that direct the pharmaceutical composition to be administered subcutaneously at a dose of 60 to 300 mg and at intervals of 1 to 8 weeks in order to treat a patient having TNF-α-related disease.

30. Use of an anti-TNF-α antibody or its antigen binding fragment in preparation of a pharmaceutical composition to be administered subcutaneously to a patient in order to treat TNF-α-related disease, wherein the anti-TNF-α antibody or its antigen binding fragment is to be administered subcutaneously at a dose of 60 to 300 mg and at intervals of 1 to 8 weeks.
